# EUROPEAN PATENT APPLICATION

(11) **EP 4 516 902 A1**
(43) Date of publication of application: **05.03.2025**
(21) Application number: 23796347.5
(22) Date of filing: 25.04.2023
(51) Int. Cl.: C12N 15/11, C12N 5/10, C12N 15/13, C12N 15/63, C12P 21/02, C12P 21/08

(54) **PROMOTER OF ENO1 GENE**

(30) Priority: 26.04.2022 JP 2022072103; 06.10.2022 JP 2022161649
(71) Applicant: Daiichi Sankyo Company, Limited, Tokyo 103-8426 (JP)
(72) Inventor: NISHIO Maui, Tokyo 103-8426 (JP); NAKAZAWA Yuto, Tokyo 103-8426 (JP); KAMIZONO Shotaro, Tokyo 103-8426 (JP)
(74) Representative: Vossius & Partner Patentanwälte Rechtsanwälte mbB
(86) International application number: PCT/JP2023/016191
(87) International publication number: WO 2023/210607

(57) **Abstract**

Provided is a promoter having high foreign gene expression-enhancing activity in host cells such as cultured mammalian cells. An approach for using the promoter to enhance the production of a foreign protein to be used as a protein-based pharmaceutical product is the provision of a polynucleotide that is a Chinese hamster-derived gene promoter, the polynucleotide comprising a nucleotide sequence that is selected from SEQ ID NO: 1, SEQ ID NO: 3, SEQ ID NO: 4, SEQ ID NO: 10, SEQ ID NO: 11, SEQ ID NO: 12, SEQ ID NO: 13 and SEQ ID NO: 14, or has 85% or higher sequence identity to each of the sequences, or a partial sequence of the nucleotide sequence.

## Description

### Technical Field

The present invention relates to transfected mammalian cells with enhanced transcriptional activity in relation to a foreign protein, which is obtained by using a foreign gene expression vector having an Enol gene promoter, and a method for producing the foreign protein using the same.

### Background Art

The development of genetic engineering techniques has rapidly expanded the market of protein-based pharmaceutical products such as therapeutic proteins and antibody drugs. Among them, antibody drugs are considered to have little risk of causing adverse immune responses when administered to the human body and are under active development because of their high specificity.

Examples of hosts to produce the protein-based pharmaceutical products typified by antibody drugs can include microorganisms, yeasts, animal and plant cells, insects, and transgenic animals and plants. Posttranslational modification such as correct folding or sugar chain modification is often essential for the physiological activity or antigenicity of the protein-based pharmaceutical products. Therefore, microorganisms which cannot perform sugar modification, or plants which differ significantly in sugar chain structure from humans are not suitable as hosts. Cultured mammalian cells, such as CHO (Chinese hamster ovary) cells are currently mainstream, which can produce the protein-based pharmaceutical products possessing a sugar chain structure similar to that of humans, permit posttranslational modification, and are confirmed to cause no safety concern from their many usage records.

Use of cultured mammalian cells as a host presents problems such as low growth rates, low productivity and high cost as compared with microorganisms or the like (Non Patent Literature 1). Furthermore, clinical utilization of antibody pharmaceutical products requires administering the pharmaceutical products at large doses. Therefore, a lack of sufficient production capacity thereof has been a global issue. In the case of producing a protein-based pharmaceutical product in a cultured mammalian cell expression system, reduction in production cost has been attempted by making improvements to each production step, because the production cost is higher than that of synthetic low-molecular weight pharmaceutical products. However, increasing the amount of protein produced in the cultured mammalian cell expression system is also a promising method for reduction in production cost (Non Patent Literatures 2 and 3). Accordingly, in order to increase the productivity of a foreign protein in cultured mammalian cells, many approaches such as promoters, enhancers, drug selection markers, gene amplification and culture engineering approaches have been practiced so far through trial and error.

In the case of using CHO cells as host cells, a human cytomegalovirus major immediate early promoter (hereinafter, referred to as a CMV promoter) derived from a virus is generally used for the expression of foreign genes, i.e., the production of protein-based pharmaceutical products (Non Patent Literatures 4, 5 and 6). It is also known that a polynucleotide upstream of the transcription start site of the gene (promoter region) of elongation factor-1 alpha (EF1α) (Patent Literature 1 and Non Patent Literature 7) or the human ribosomal protein RPL32 or RPS11 can be used alone or in combination with an additional heterologous promoter in protein expression in CHO cells (Non Patent Literature 8 and Patent Literatures 2 and 3). The heat-shock protein A5 (Hspa5/GRP78) gene promoter and the heat shock protein A8 (Hspa8) promoter are known to improve the productivity of a foreign protein in mammalian cells (Patent Literatures 4 and 5).

### Citation List

### Patent Literature

Patent Literature 1: Japanese Patent No. 3051411
Patent Literature 2: International Publication No. WO 2006/123097
Patent Literature 3: International Publication No. 2013/080934
Patent Literature 4: International Publication No. 2018/066492
Patent Literature 5: International Publication No. 2020/032153

### Non Patent Literature

Non Patent Literature 1: Florian M. Wurm., Nat. Biotechnol. 22 (11): 1393-1398, 2004
Non Patent Literature 2: Farid SS., J Chromatogr B Analyt Technol Biomed Life Sci. 848 (1): 8-18, 2007
Non Patent Literature 3: Werner RG. Economic aspects of commercial manufacture of biopharmaceuticals. J Biotechnol. 113 (1-3): 171-182, 2004
Non Patent Literature 4: Durocher Y et al., Curr Opin Biotechnol. 20 (6): 700-707, 2009
Non Patent Literature 5: Boshart M et al., Cell. 41 (2): 521-530, 1985
Non Patent Literature 6: Foecking MK et al., Gene. 45 (1): 101-105, 1986
Non Patent Literature 7: Deer JR. and Allison DS., Biotechnol. Prog. 20: 880-889, 2004
Non Patent Literature 8: Hoeksema F. et al., Biotechnology Research International, Volume 2011, Article ID 492875, 11 pages

### Summary of Invention

### Technical Problem

An object of the present invention is to provide a promoter having high foreign gene expression-enhancing activity in host cells such as cultured mammalian cells, and to provide an approach to enhancing the amount of a foreign protein, which serves as a protein-based pharmaceutical product, produced using the promoter.

### Solution to Problem

For solving the above-described problems, the present invention provides the following [1] to [34].
[1] A polynucleotide that is a Chinese hamster-derived gene promoter, the polynucleotide comprising a nucleotide sequence that is selected from SEQ ID NO: 1, SEQ ID NO: 3, SEQ ID NO: 4, SEQ ID NO: 10, SEQ ID NO: 11, SEQ ID NO: 12, SEQ ID NO: 13 and SEQ ID NO: 14, or has 85% or higher sequence identity to each of the sequences, or a partial sequence of the nucleotide sequence.
[2] The polynucleotide according to [1], which comprises a nucleotide sequence that is selected from SEQ ID NO: 1, SEQ ID NO: 3, SEQ ID NO: 4, SEQ ID NO: 10, SEQ ID NO: 11 and SEQ ID NO: 12, or has 85% or higher sequence identity to each of the sequences, or a partial sequence of the nucleotide sequence.
[2a] The polynucleotide according to [1] or [2], which comprises a nucleotide sequence of SEQ ID NO: 18, or a nucleotide sequence having 85% or higher sequence identity to the sequence.
[3] The polynucleotide of according to [1], which consists of a nucleotide sequence having 99% or higher sequence identity to the nucleotide sequence of SEQ ID NO: 1.
[4] The polynucleotide of according to [1], which consists of a nucleotide sequence having 99% or higher sequence identity to the nucleotide sequence of SEQ ID NO: 3.
[5] The polynucleotide of according to [1], which consists of a nucleotide sequence having 99% or higher sequence identity to the nucleotide sequence of SEQ ID NO: 4.
[6] The polynucleotide of according to [1], which consists of a nucleotide sequence having 99% or higher sequence identity to the nucleotide sequence of SEQ ID NO: 10.
[7] The polynucleotide of according to [1], which consists of a nucleotide sequence having 99% or higher sequence identity to the nucleotide sequence of SEQ ID NO: 11.
[8] The polynucleotide of according to [1], which consists of a nucleotide sequence having 99% or higher sequence identity to the nucleotide sequence of SEQ ID NO: 12.
[9] The polynucleotide of according to [1], which consists of a nucleotide sequence having 99% or higher sequence identity to the nucleotide sequence of SEQ ID NO: 13.
[10] The polynucleotide of according to [1], which consists of a nucleotide sequence having 99% or higher sequence identity to the nucleotide sequence of SEQ ID NO: 14.
   [10a] A polynucleotide that is a Chinese hamster-derived gene promoter, the polynucleotide comprising a nucleotide sequence of SEQ ID NO: 18, or a nucleotide sequence having 85% or higher sequence identity to the sequence.
   [10b] The polynucleotide according to [10a], which comprises a nucleotide sequence that is selected from SEQ ID NO: 1, SEQ ID NO: 3, SEQ ID NO: 4, SEQ ID NO: 10, SEQ ID NO: 11, SEQ ID NO: 12 and SEQ ID NO: 13, or has 85% or higher sequence identity to each of the sequences, or a partial sequence of the nucleotide sequence.
[11] A polynucleotide that is a mouse-derived gene promoter, the polynucleotide comprising a nucleotide sequence that is selected from SEQ ID NO: 2, SEQ ID NO: 17 and SEQ ID NO: 16, or has 85% or higher sequence identity to each of the sequences, or a partial sequence of the nucleotide sequence.
[11a] The polynucleotide according to [11], which comprises a nucleotide sequence having 85% or higher sequence identity to the nucleotide sequence of SEQ ID NO: 18.
[12] The polynucleotide according to [11], which consists of a nucleotide sequence having 99% or higher sequence identity to the nucleotide sequence of SEQ ID NO: 2.
[13] The polynucleotide according to [11], which consists of a nucleotide sequence having 99% or higher sequence identity to the nucleotide sequence of SEQ ID NO: 17.
[14] The polynucleotide according to [11], which consists of a nucleotide sequence having 99% or higher sequence identity to the nucleotide sequence of SEQ ID NO: 16.
   [14a] A polynucleotide that is a mouse-derived gene promoter, the polynucleotide comprising a nucleotide sequence having 85% or higher sequence identity to the nucleotide sequence of SEQ ID NO: 18.
   [14b] The polynucleotide according to [14a], which comprises a nucleotide sequence that is selected from SEQ ID NO: 2, SEQ ID NO: 17 and SEQ ID NO: 16, or has 85% or higher sequence identity to each of the sequences, or a partial sequence of the nucleotide sequence.
[15] A polynucleotide comprising a nucleotide sequence having 90% or higher sequence identity to the nucleotide sequence of SEQ ID NO: 1, SEQ ID NO: 3, SEQ ID NO: 4, SEQ ID NO: 10, SEQ ID NO: 11, SEQ ID NO: 12, SEQ ID NO: 13, SEQ ID NO: 14, SEQ ID NO: 2, SEQ ID NO: 17 or SEQ ID NO: 16 in [1] or [11], or a partial sequence of the nucleotide sequence, the polynucleotide having promoter activity.
[16] A polynucleotide comprising a nucleotide sequence having 95% or higher sequence identity to the nucleotide sequence of SEQ ID NO: 1, SEQ ID NO: 3, SEQ ID NO: 4, SEQ ID NO: 10, SEQ ID NO: 11, SEQ ID NO: 12, SEQ ID NO: 13, SEQ ID NO: 14, SEQ ID NO: 2, SEQ ID NO: 17 or SEQ ID NO: 16 in [1] or [11], or a partial sequence of the nucleotide sequence, the polynucleotide having promoter activity.
[17] A polynucleotide that hybridizes under stringent conditions to a polynucleotide consisting of a nucleotide sequence complementary to the nucleotide sequence of SEQ ID NO: 1, SEQ ID NO: 3, SEQ ID NO: 4, SEQ ID NO: 10, SEQ ID NO: 11, SEQ ID NO: 12, SEQ ID NO: 13, SEQ ID NO: 14, SEQ ID NO: 2, SEQ ID NO: 17 or SEQ ID NO: 16 in [1] or [11], the polynucleotide having promoter activity.
[17a] The polynucleotide according to any one of [1] to [17], which has a length of 1 kbp or more and 10 kbp or less, 2 kbp or more and 6 kbp or less, 2.6 kbp or more and 5 kbp or less, 2.7 kbp or more and 4.5 kbp or less, or 2.8 kbp or more and 4 kbp or less.
[18] A polynucleotide comprising the polynucleotide according to any one of [1] to [17] and [17a] and a foreign gene.
[19] A foreign gene expression unit comprising the polynucleotide according to any one of [1] to [18] and [17a] and a foreign gene.
[19a] The foreign gene expression unit according to [19], which further comprises a transcriptional terminator region.
[20] The foreign gene expression unit according to [19] or [19a], wherein the foreign gene is a gene encoding a protein.
[21] The foreign gene expression unit according to [20], wherein the foreign gene is a gene encoding a heteromultimeric protein.
[22] The foreign gene expression unit according to [21], wherein the foreign gene is a gene encoding an antibody or an antigen-binding fragment thereof.
[23] A foreign gene expression vector comprising the foreign gene expression unit according to any one of [19] to [22] and [19a].
[24] A foreign gene expression vector comprising the foreign gene expression unit according to any one of [19] to [22] and [19a], and one or more polynucleotides selected from the following (a) to (e):
   (a) a polynucleotide comprising the nucleotide sequence of SEQ ID NO: 5, or a partial sequence thereof;
   (b) a polynucleotide comprising the nucleotide sequence of SEQ ID NO: 6, or a partial sequence thereof;
   (c) a polynucleotide comprising the nucleotide sequence of SEQ ID NO: 7, or a partial sequence thereof;
   (d) a polynucleotide comprising a nucleotide sequence having 80% or higher sequence identity to the nucleotide sequence of SEQ ID NO: 5, SEQ ID NO: 6 or SEQ ID NO: 7, or a partial sequence of the nucleotide sequence, the polynucleotide having foreign gene expression-enhancing activity; and
   (e) a polynucleotide comprising a nucleotide sequence having 85% or higher sequence identity to the nucleotide sequence of SEQ ID NO: 5, SEQ ID NO: 6 or SEQ ID NO: 7, or a partial sequence of the nucleotide sequence, the polynucleotide having foreign gene expression-enhancing activity.
[24a] A foreign gene expression vector comprising the foreign gene expression unit according to any one of [19] to [22] and [19a], and the following polynucleotide (b) or (d):
   (b) a polynucleotide comprising the nucleotide sequence of SEQ ID NO: 6, or a partial sequence thereof; or
   (d) a polynucleotide comprising a nucleotide sequence having 80% or higher identity to the nucleotide sequence of SEQ ID NO: 6, or a partial sequence of the nucleotide sequence, the polynucleotide having foreign gene expression-enhancing activity.
[25] A transfected cell into which the foreign gene expression vector according to [23], [24] or [24a] has been introduced.
[26] A transfected cell according to [24], wherein the cell is a cultured cell derived from a mammal.
[27] The transfected cell according to [25], wherein the cell is a COS-1 cell, a 293 cell, or a CHO cell.
[28] A method for producing a foreign gene-derived protein, comprising culturing the transfected cell according to any one of [25] to [27], and obtaining the foreign gene-derived protein from the culture.
[29] The production method according to [28], wherein the foreign gene-derived protein is a monomeric protein or a multimeric protein.
[30] The production method according to [29], wherein the multimeric protein is a heteromultimeric protein.
[31] The production method according to [30], wherein the heteromultimeric protein is an antibody protein.
[31a] The production method according to any one of [28] to [31], wherein the culture of the transfected cell is fed-batch culture.
[31b] The production method according to any one of [28] to [31] and [31a], wherein the transfected cell is cultured for 6 or more days.
[32] Use of the polynucleotide according to any one of [1] to [18] for expressing a foreign gene in a transfected cell.
[33] Use of the foreign gene expression vector according to [23] or [24] for the purpose of expressing a foreign gene in a transfected cell.
[34] A method for producing a transfected cell which expresses a foreign gene, the method comprising performing transfection with the foreign gene vector according to [23], [24] or [24a].

### Advantageous Effects of the Invention

The present invention provides a promoter having high foreign gene expression-enhancing activity in host cells such as cultured mammalian cells, and an approach to enhancing the amount of a foreign protein, which serves as a protein-based pharmaceutical product, produced using the promoter.

### Brief Description of Drawings

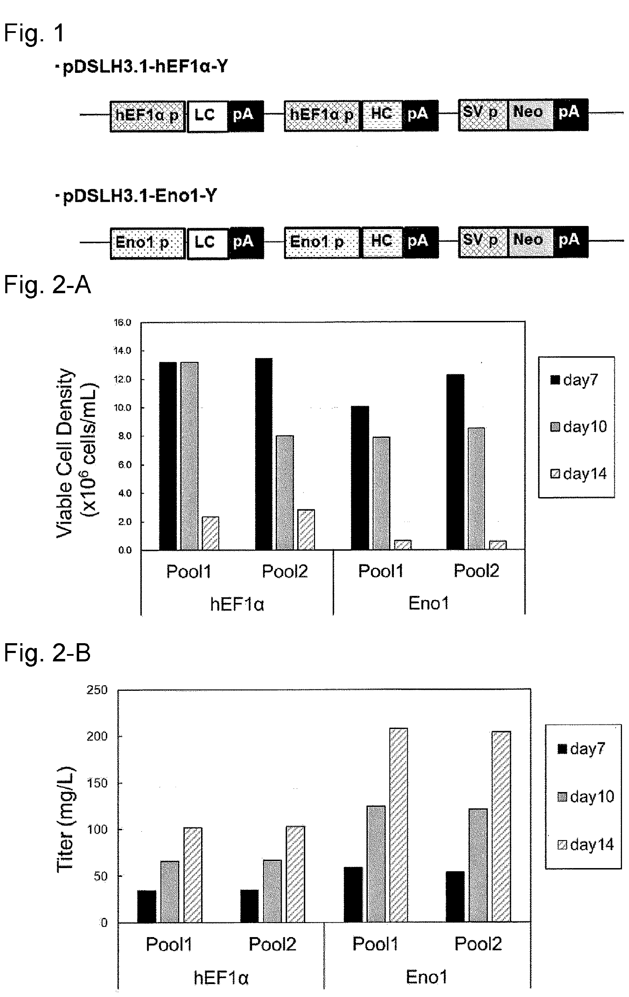

[Figure 1] Figure 1 schematically shows humanized antibody gene Y expression vectors pDSLH3.1-Eno1-Y and pDSLH3.1-hEF1α-Y containing an Enol gene or human EF1α gene-derived promoter as a promoter for antibody H chain (HC) and L chain (LC) gene expression.
[Figure 2-A] The amount of antibody produced in fed-batch culture using a humanized antibody Y-expressing stable pool was compared between expression under a Chinese hamster-derived Enol gene promoter and expression under a human EF1-α gene promoter. In Figure 2-A, the vertical axis represents the number of viable cells on each day of sampling. hEF1α denotes a human EF1α gene promoter, and Eno1 denotes a Chinese hamster-derived Enol gene promoter (the same applies to Figure 2-B).
[Figure 2-B] The amount of antibody produced in fed-batch culture using a humanized antibody Y-expressing stable pool was compared between expression under a Chinese hamster-derived Enol gene promoter and expression under a human EF1-α gene promoter. In Figure 2-B, the vertical axis represents the amount of the antibody produced on each day of sampling.

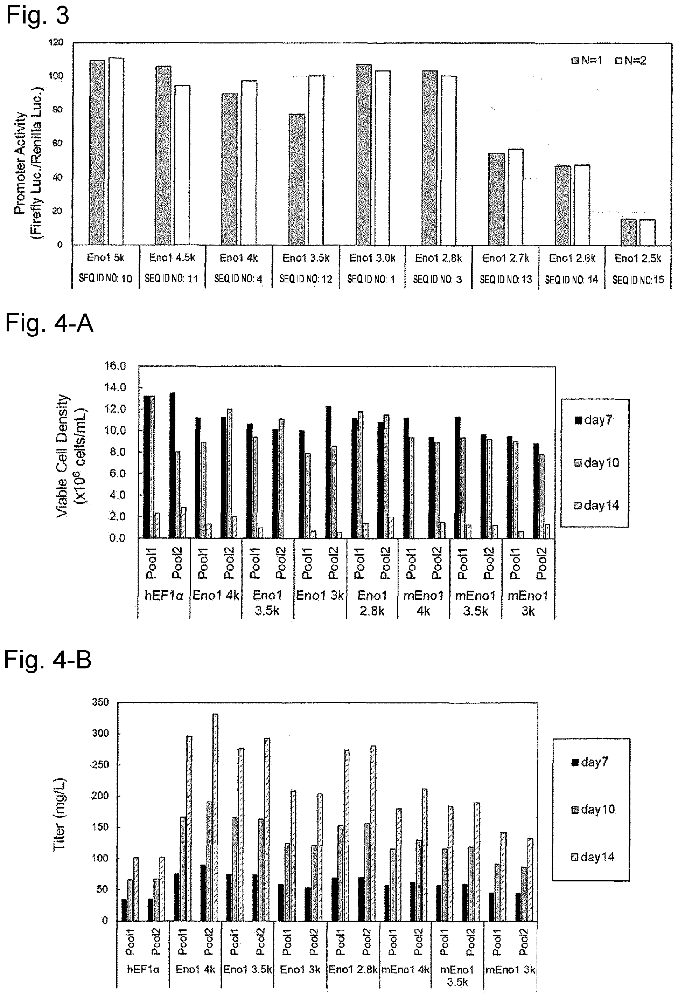

[Figure 3] The expression level of firefly luciferase transiently expressed using the region of approximately 2.5 kbp from approximately 5.0 kbp upstream from the start codon of a Chinese hamster-derived Enol gene as a promoter was compared between the chain lengths. The vertical axis represents a value obtained by dividing the amount of luminescence of firefly-derived luciferase on pGL 4.10 by the amount of luminescence of Renilla-derived luciferase on pGL 4.74. In the figure, Enol 5k, Enol 4.5k, Enol 4k, Enol 3.5k, Enol 3.0k, Enol 2.8k, Enol 2.7k, Enol 2.6k and Enol 2.5k denote, respectively, regions extending to approximately 5.0 kbp (SEQ ID NO: 10), approximately 4.5 kbp (SEQ ID NO: 11), approximately 4.0 kbp (SEQ ID NO: 4), approximately 3.5 kbp (SEQ ID NO: 12), approximately 3.0 kbp (SEQ ID NO: 1), approximately 2.8 kbp (SEQ ID NO: 3), approximately 2.7 kbp (SEQ ID NO: 13), approximately 2.6 kbp (SEQ ID NO: 14) and approximately 2.5 kbp (SEQ ID NO: 15) upstream from a nucleotide immediately upstream of the nucleotide sequence corresponding to the start codon sequence in the Chinese hamster-derived Enol gene.
[Figure 4-A] The amount of antibody produced in the fed-batch culture of a humanized antibody Y-expressing stable pool generated using an Enol gene promoter derived from several species was compared. Enol and mEno1 indicate results from the Chinese hamster-derived Enol gene promoter and the mouse-derived Enol gene promoter, respectively. In Figure 4-A, the vertical axis represents the number of viable cells on each day of sampling. In the figure, Enol 4k, Enol 3.5k, Enol 3k and Enol 2.8k denote, respectively, regions extending to approximately 4.0 kbp (SEQ ID NO: 4), approximately 3.5 kbp (SEQ ID NO: 12), approximately 3.0 kbp (SEQ ID NO: 1) and approximately 2.8 kbp (SEQ ID NO: 3) upstream from a nucleotide immediately upstream of the nucleotide sequence corresponding to the start codon sequence in the Chinese hamster-derived Enol gene. mEno1 4k, mEno1 3.5k and mEno1 3k denote, respectively, regions extending to approximately 4.0 kbp (SEQ ID NO: 16), approximately 3.5 kbp (SEQ ID NO: 17) and approximately 3.0 kbp (SEQ ID NO: 2) upstream from a nucleotide immediately upstream of the nucleotide sequence corresponding to the start codon sequence in the mouse-derived Enol gene (the same applies to Figure 4-B).
[Figure 4-B] The amount of antibody produced in the fed-batch culture of a humanized antibody Y-expressing stable pool generated using an Enol gene promoter derived from several species was compared . Enol and mEno1 indicate results from the Chinese hamster-derived Enol gene promoter and the mouse-derived Enol gene promoter, respectively. In Figure 4-B, the vertical axis represents the amount of the antibody produced on each day of sampling.

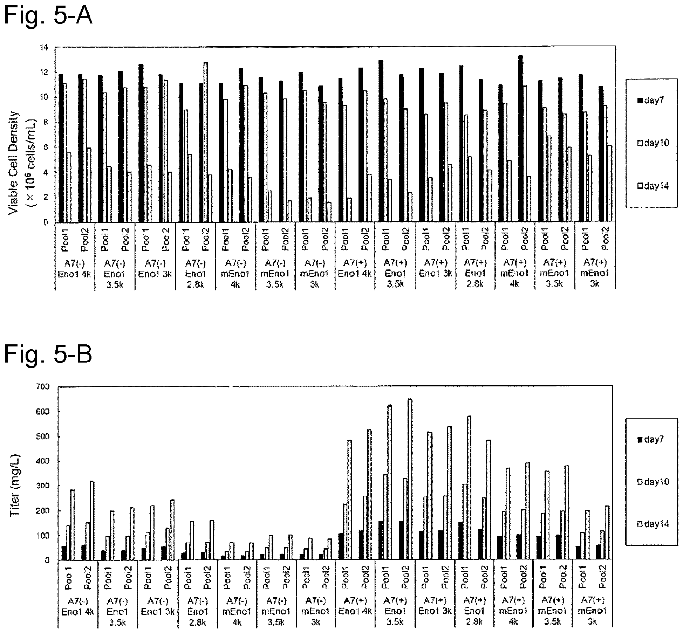

[Figure 5-A] The amount of antibody produced in the fed-batch culture of a humanized antibody Y-expressing stable pool generated using an Enol gene promoter derived from several species and DNA element A7 in combination was compared. Enol and mEno1 indicate results from the Chinese hamster-derived Enol gene promoter and the mouse-derived Enol gene promoter, respectively. In Figure 5-A, the vertical axis represents the number of viable cells on each day of sampling. In the figure, the Enol gene promoters are set similarly to those in Figure 4-A. A7(+) indicates combination with DNA element A7, and A7(-) indicates absence of A7 (the same applies to Figure 5-B).
[Figure 5-B] The amount of antibody produced in the fed-batch culture of a humanized antibody Y-expressing stable pool generated using an Enol gene promoter derived from several species and DNA element A7 in combination was compared. In Figure 5-B, the vertical axis represents the amount of the antibody produced on each day of sampling.

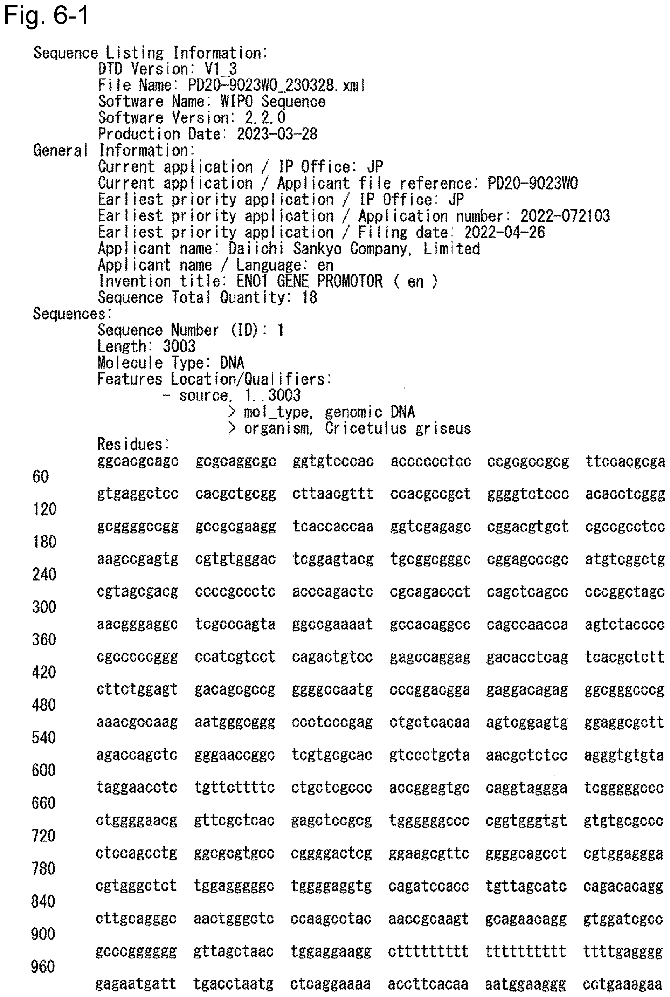

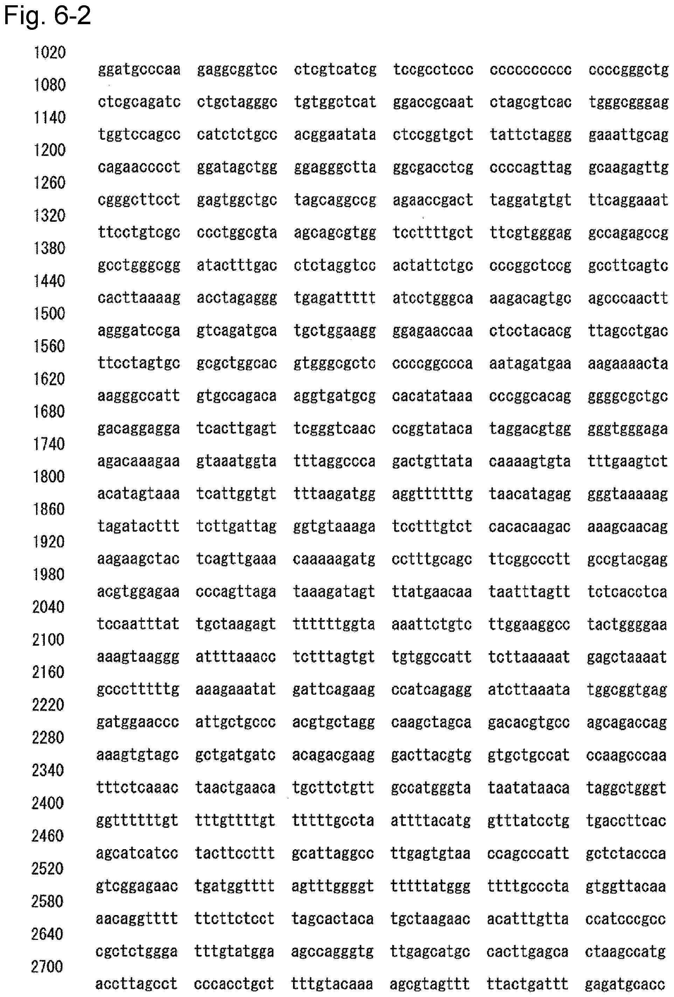

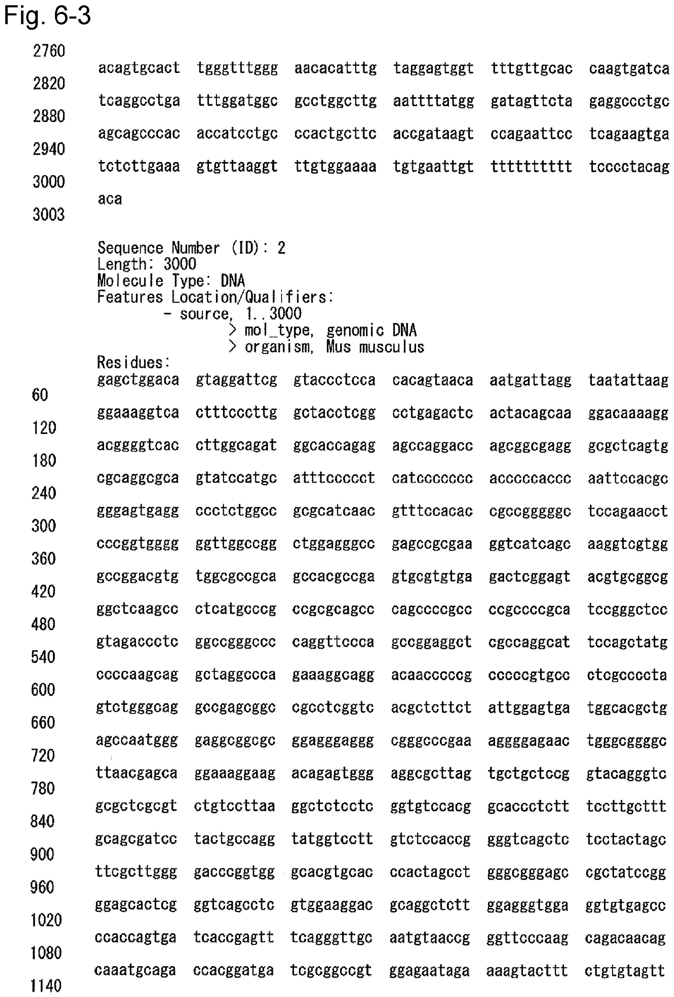

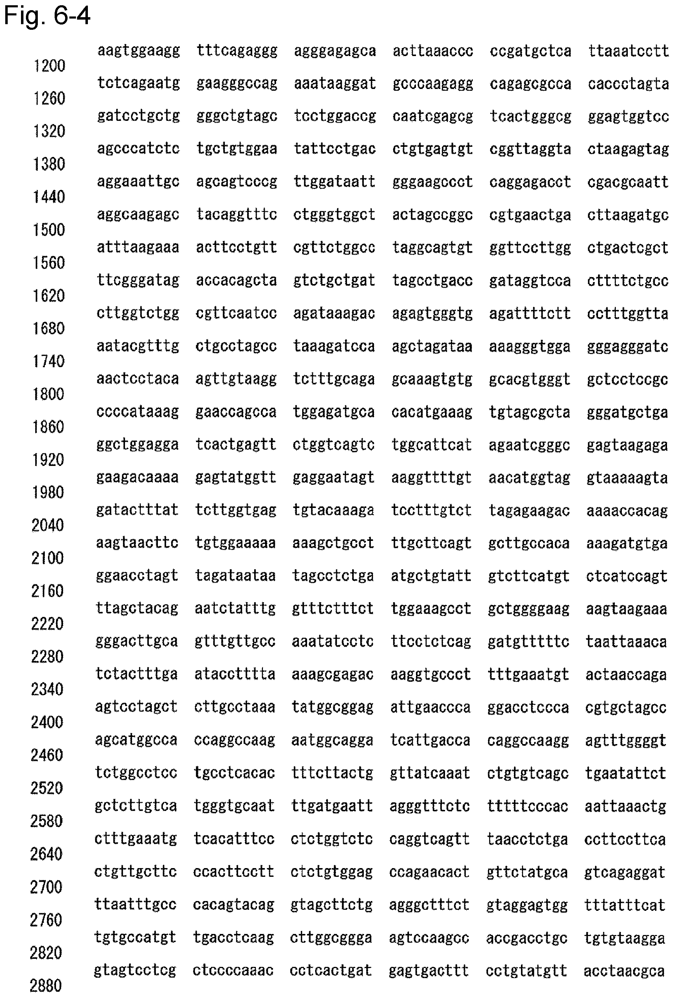

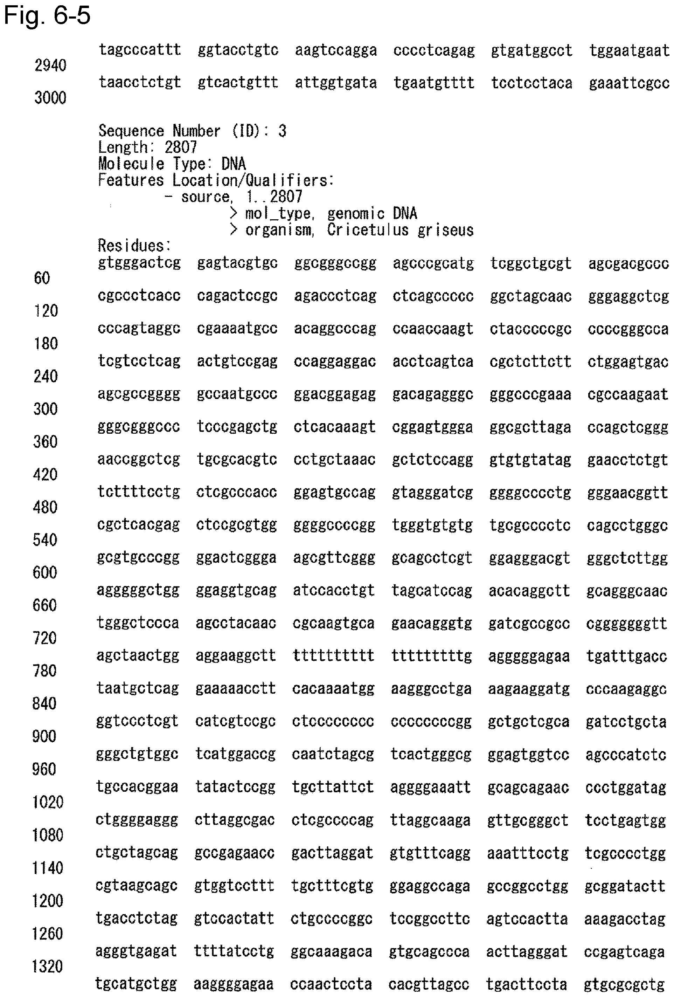

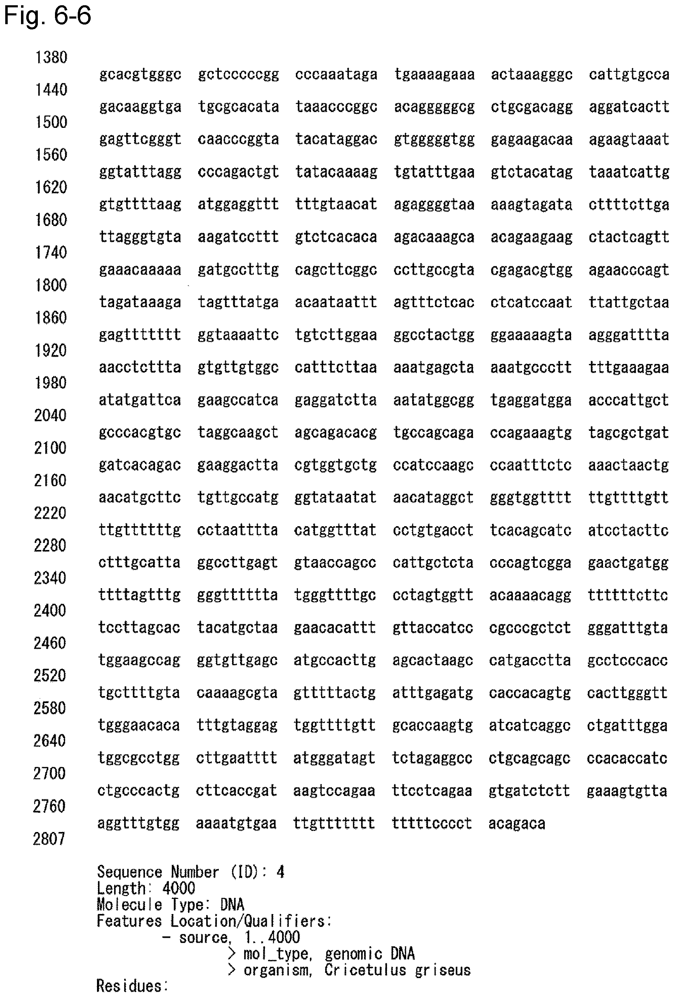

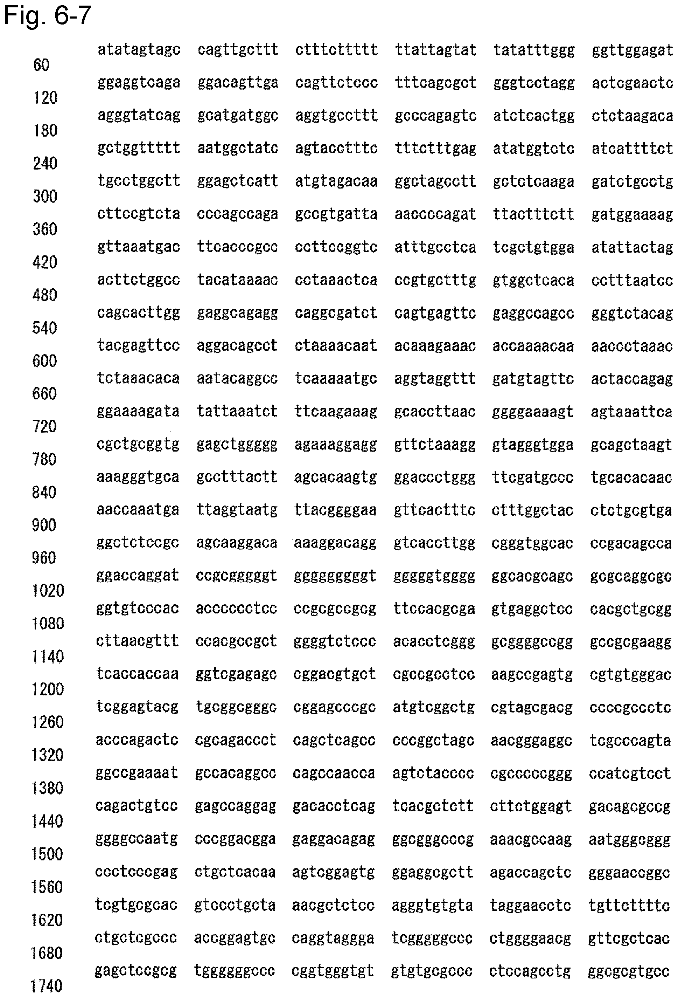

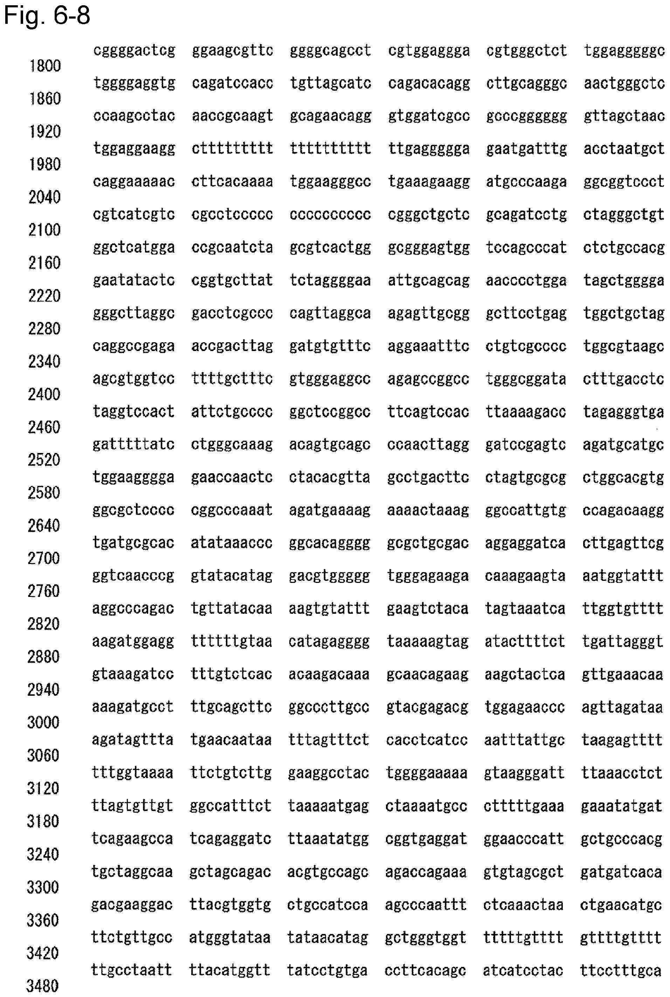

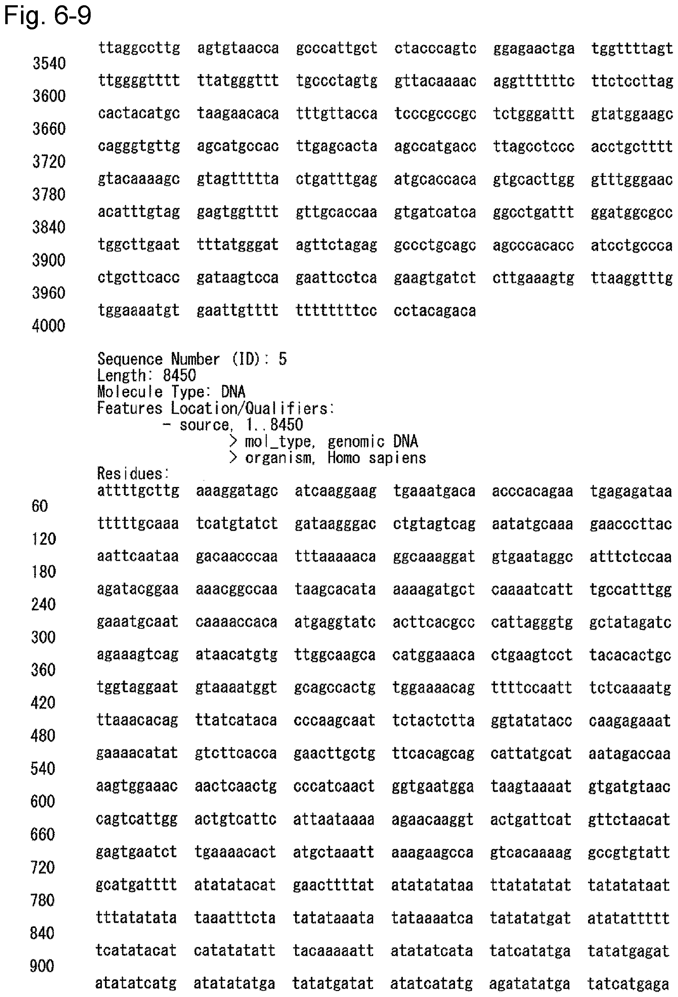

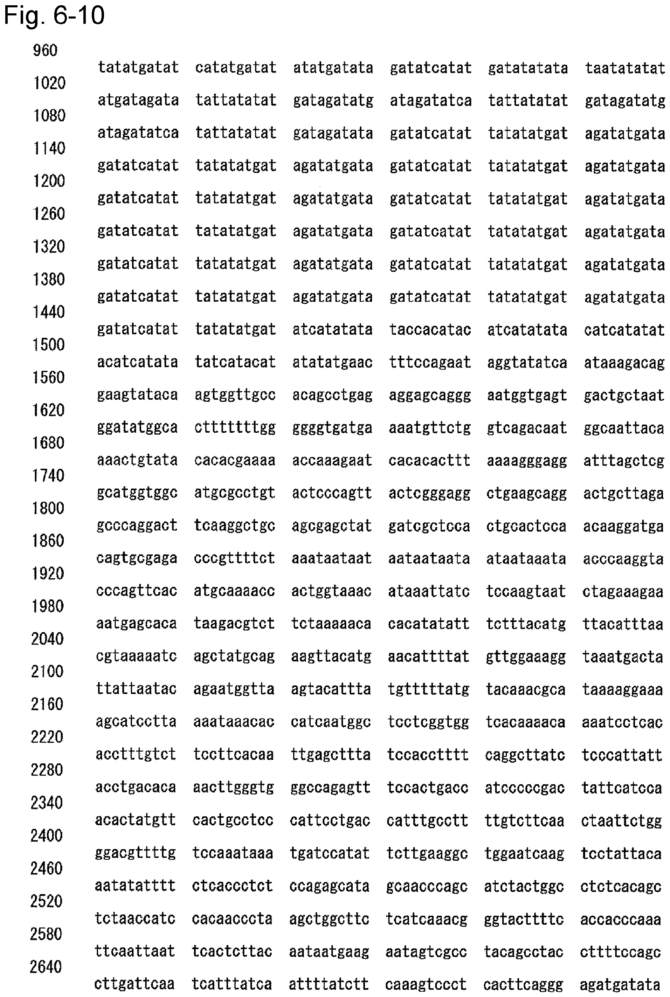

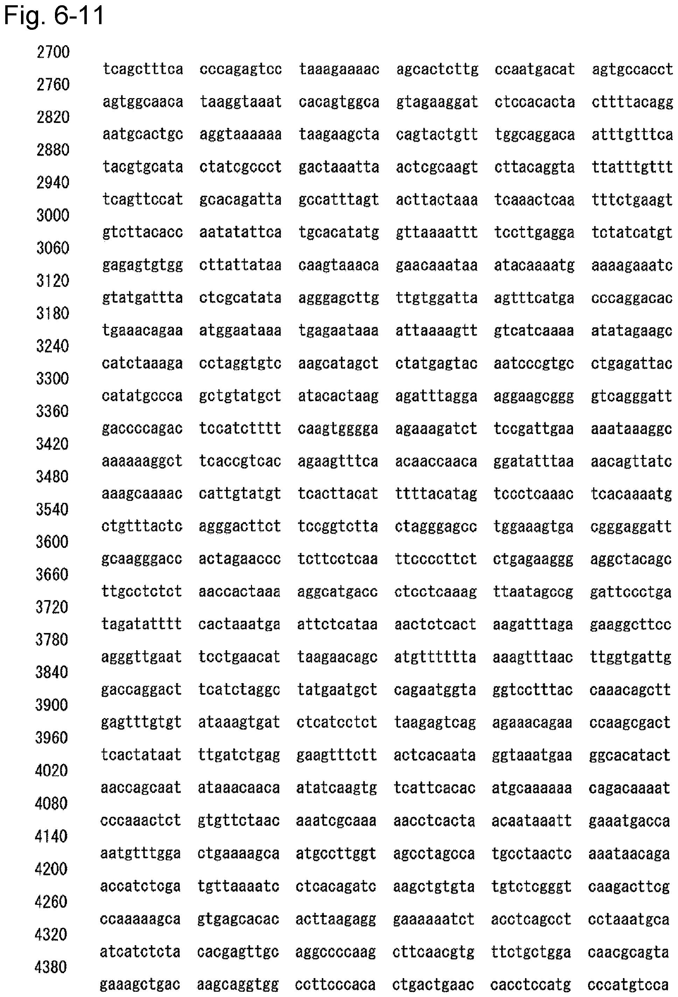

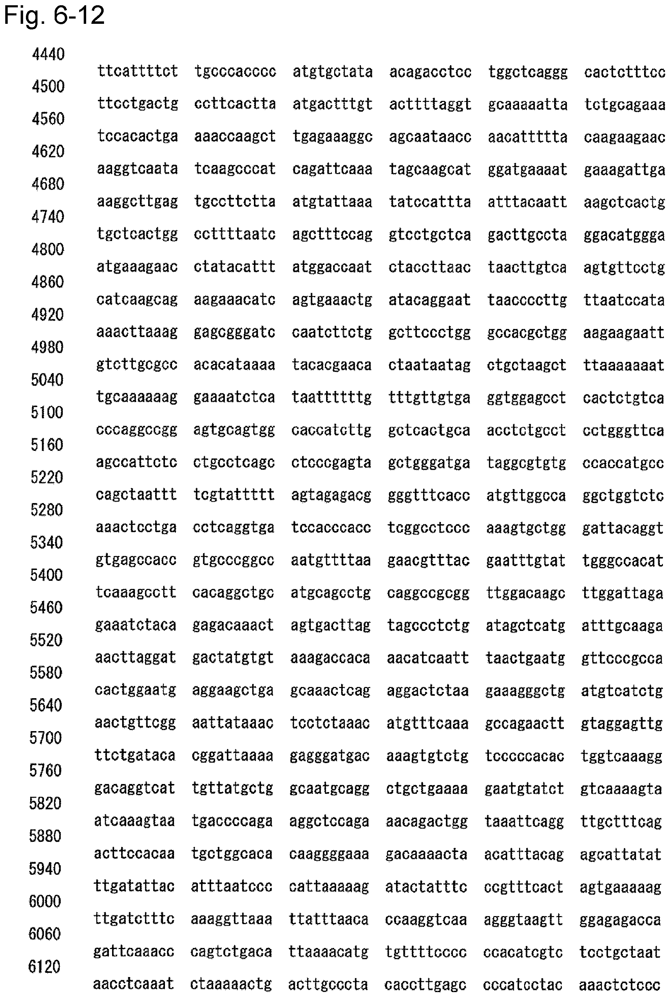

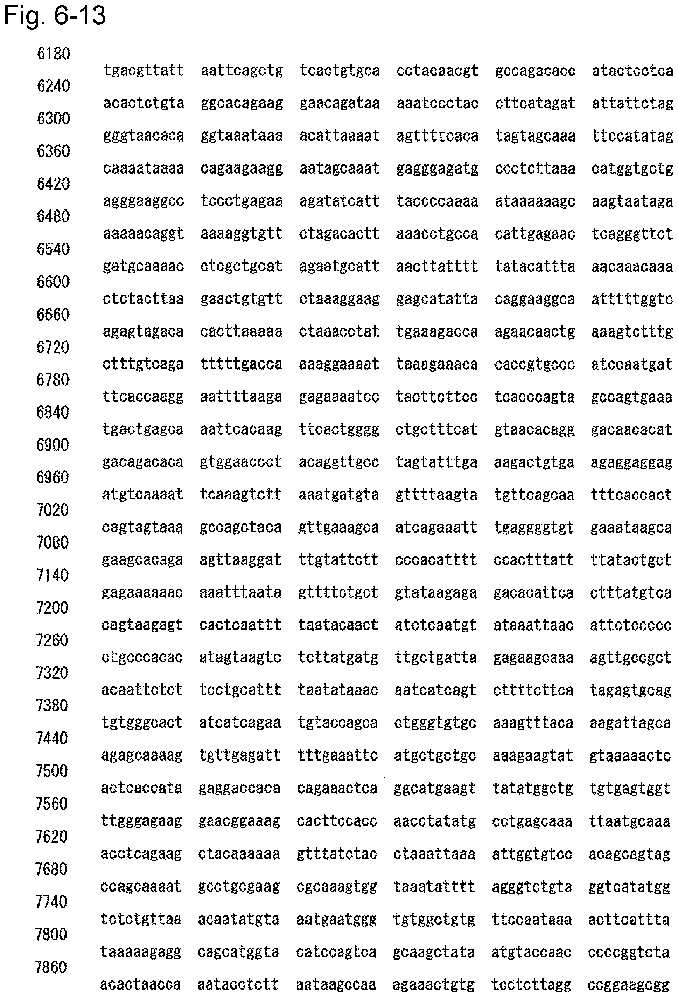

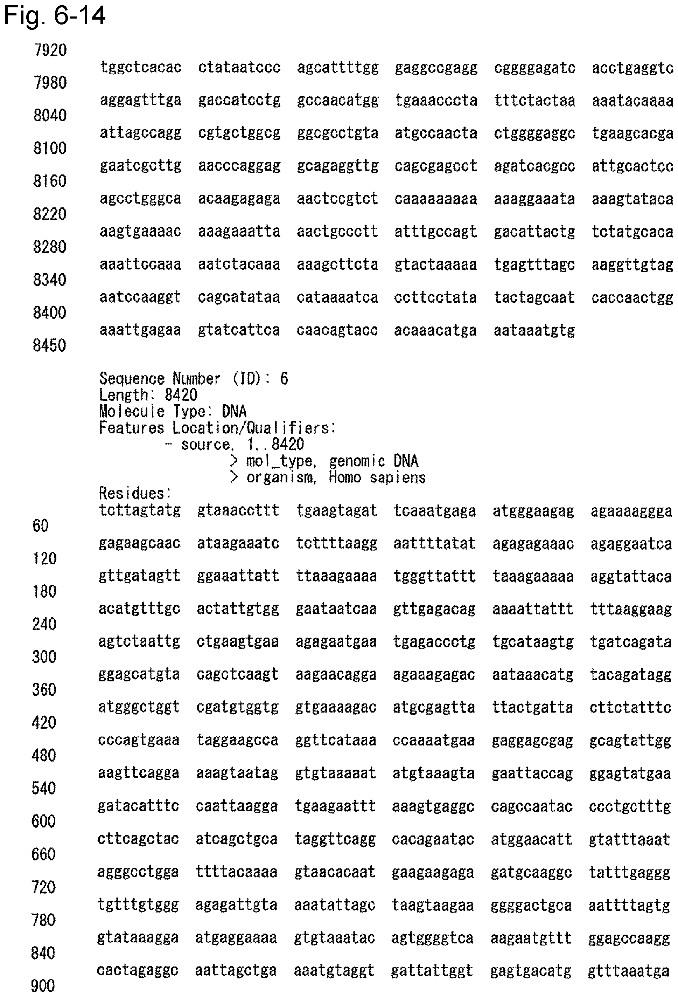

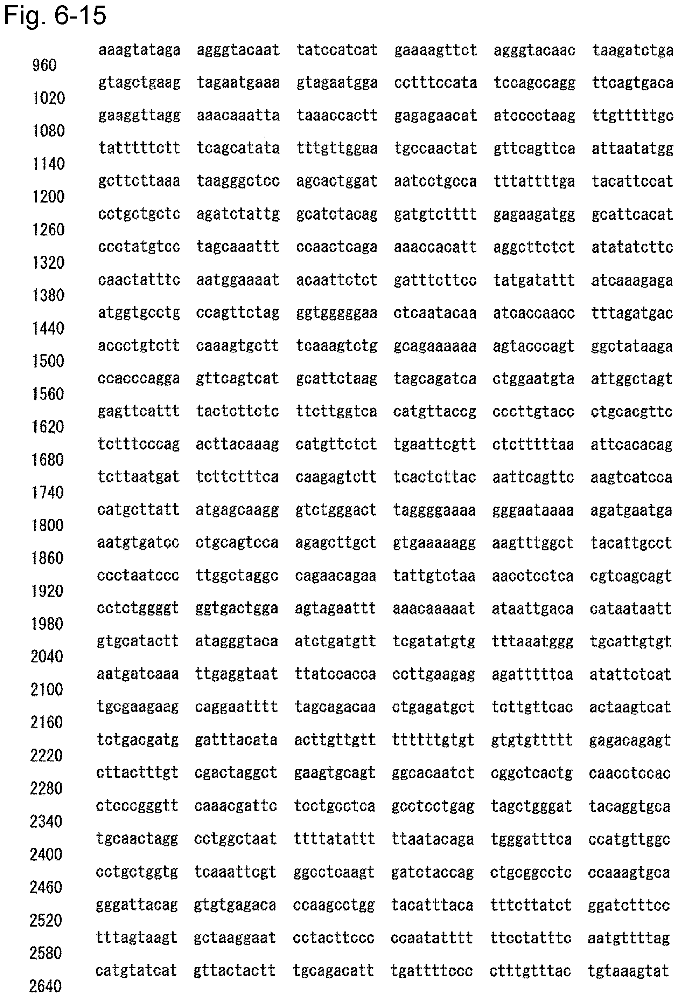

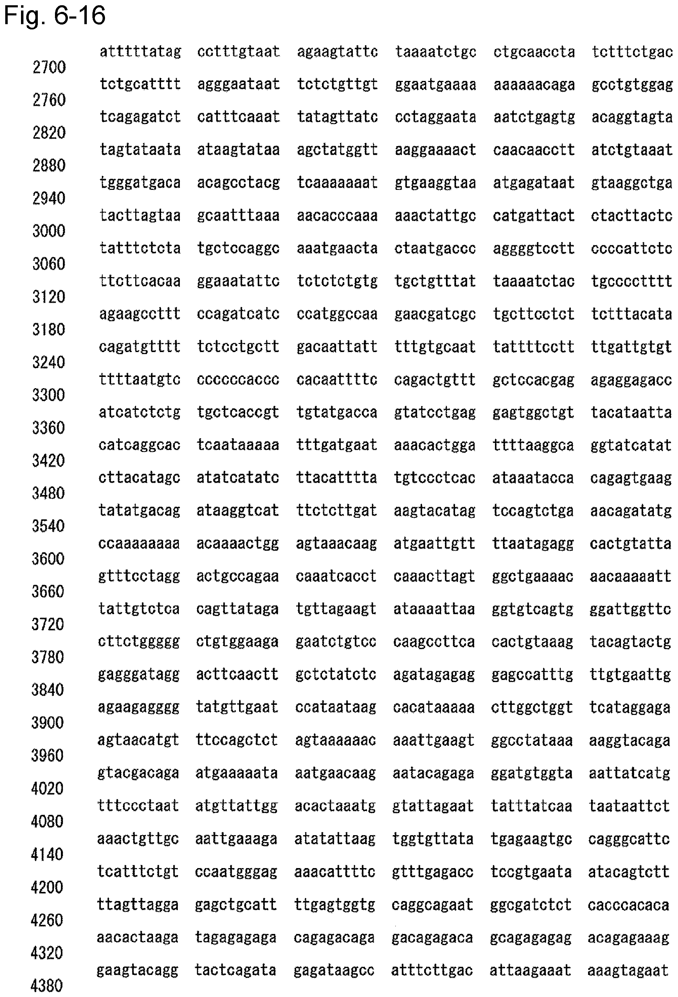

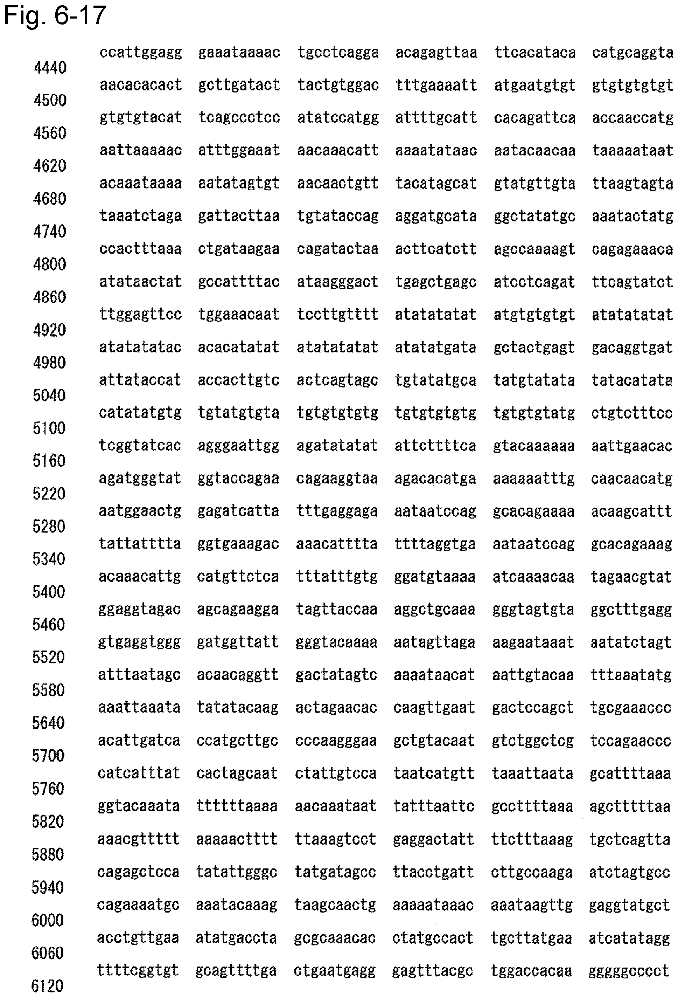

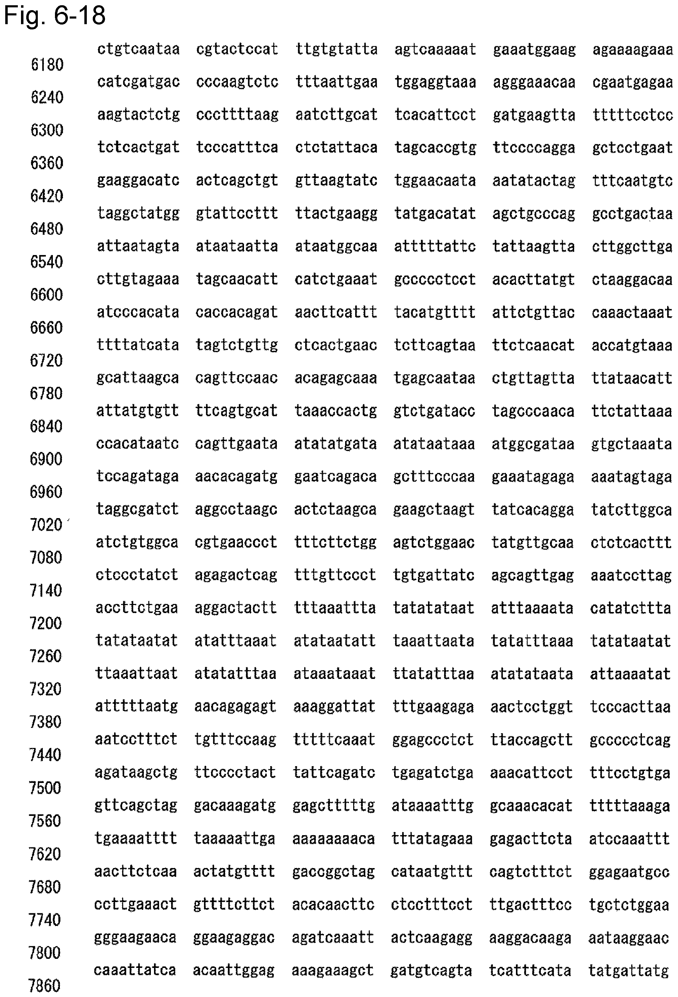

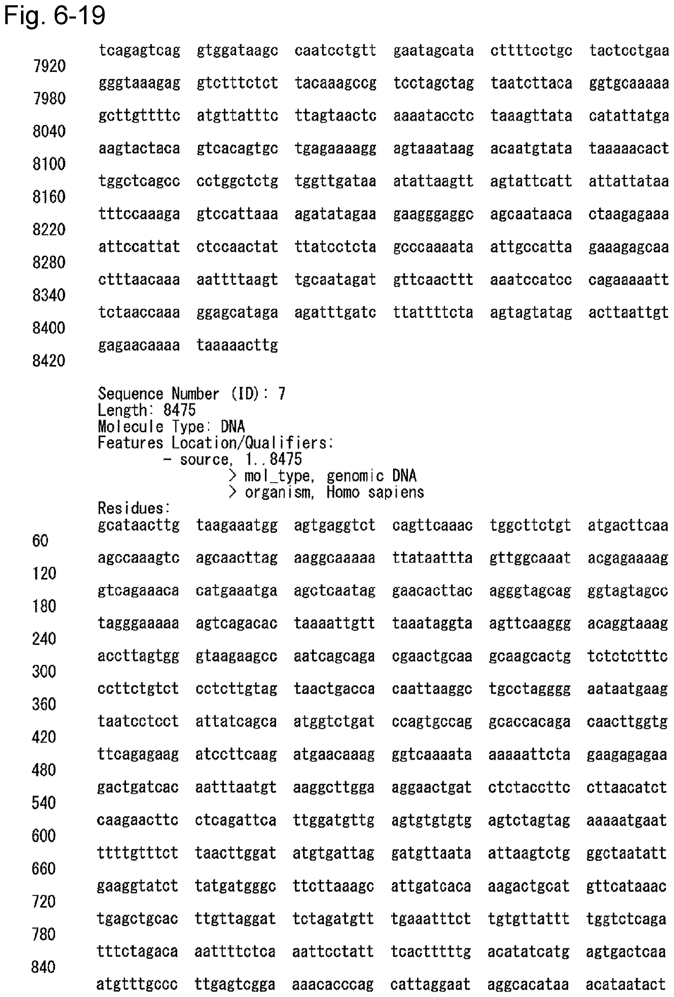

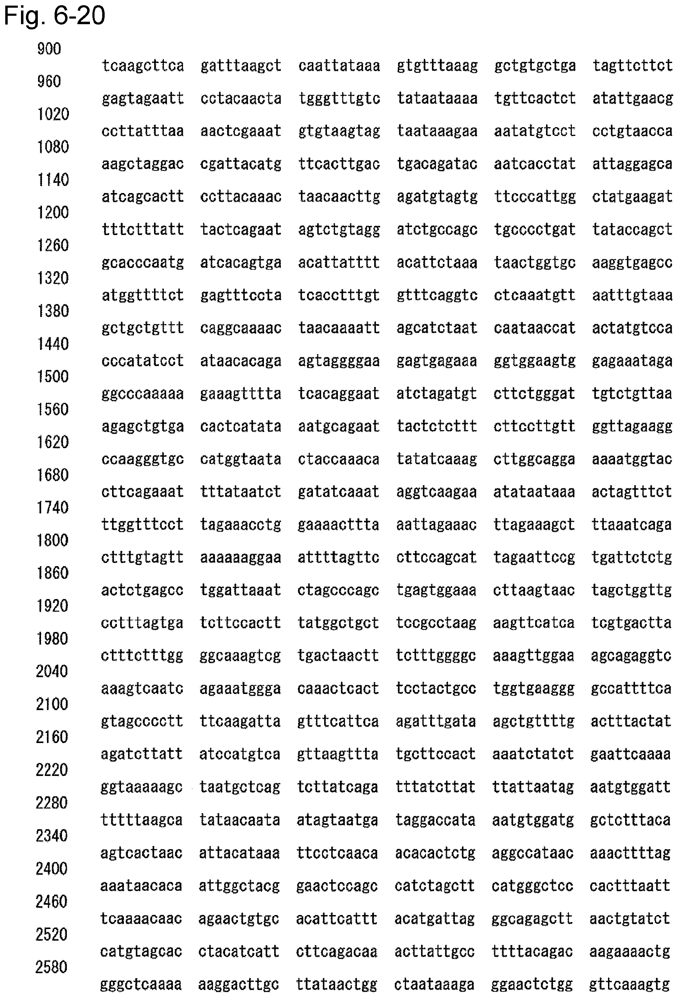

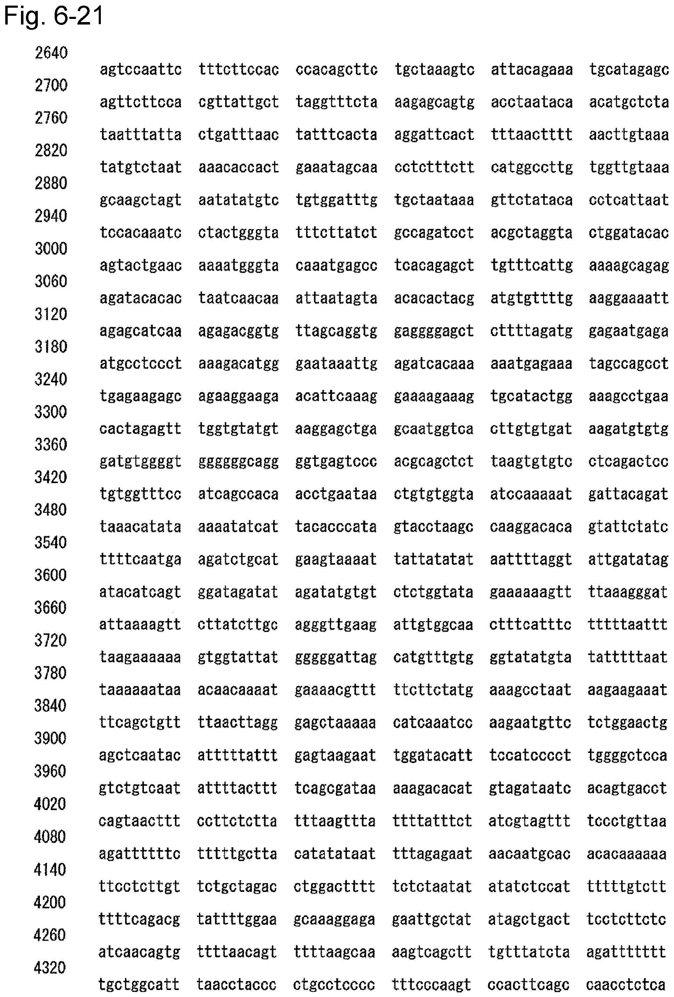

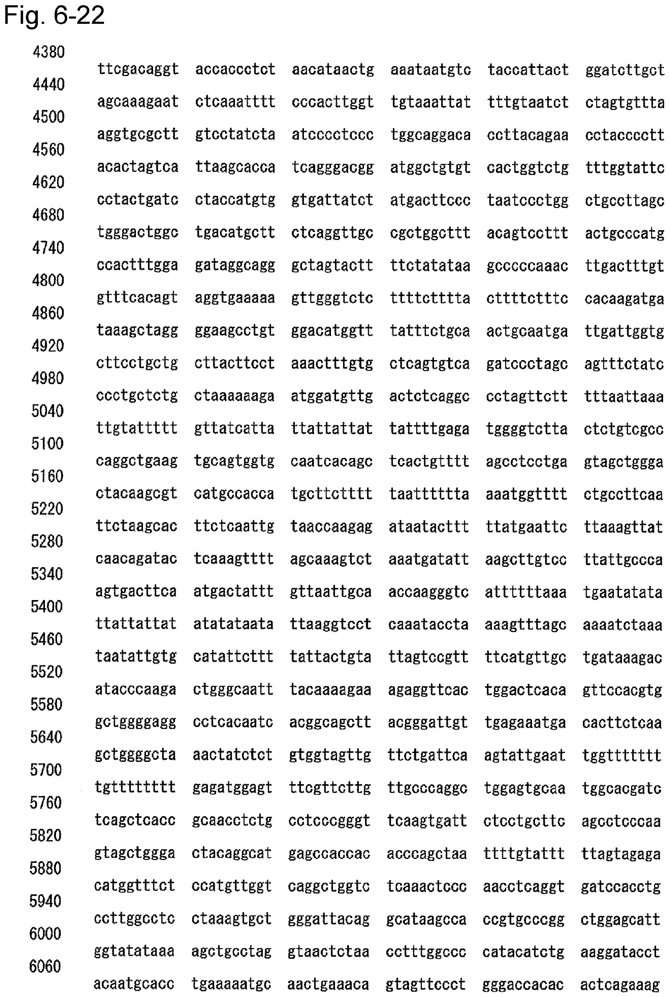

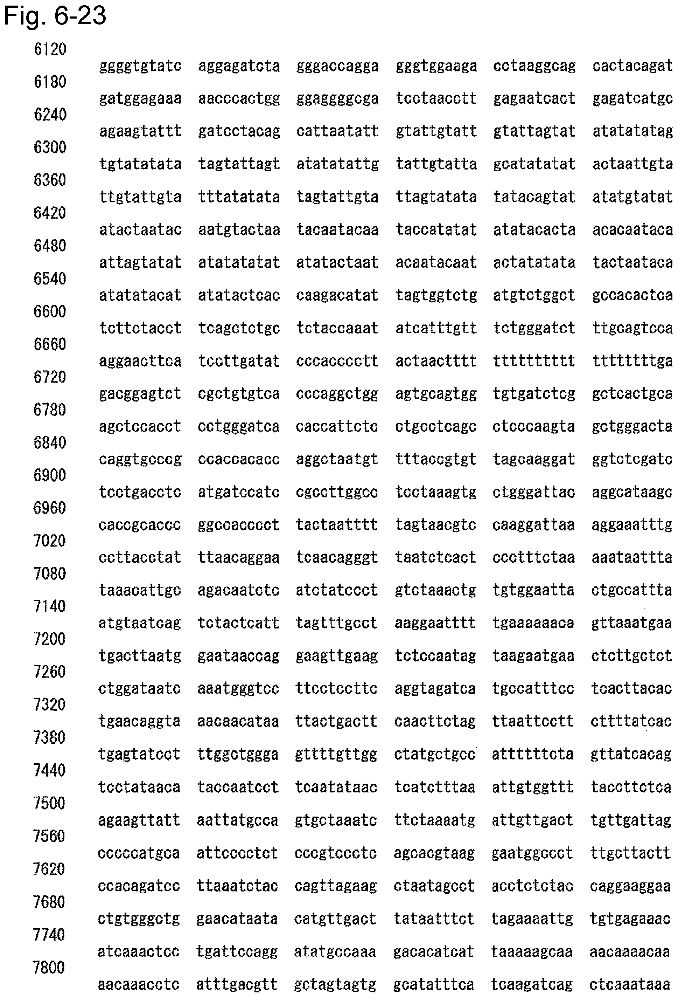

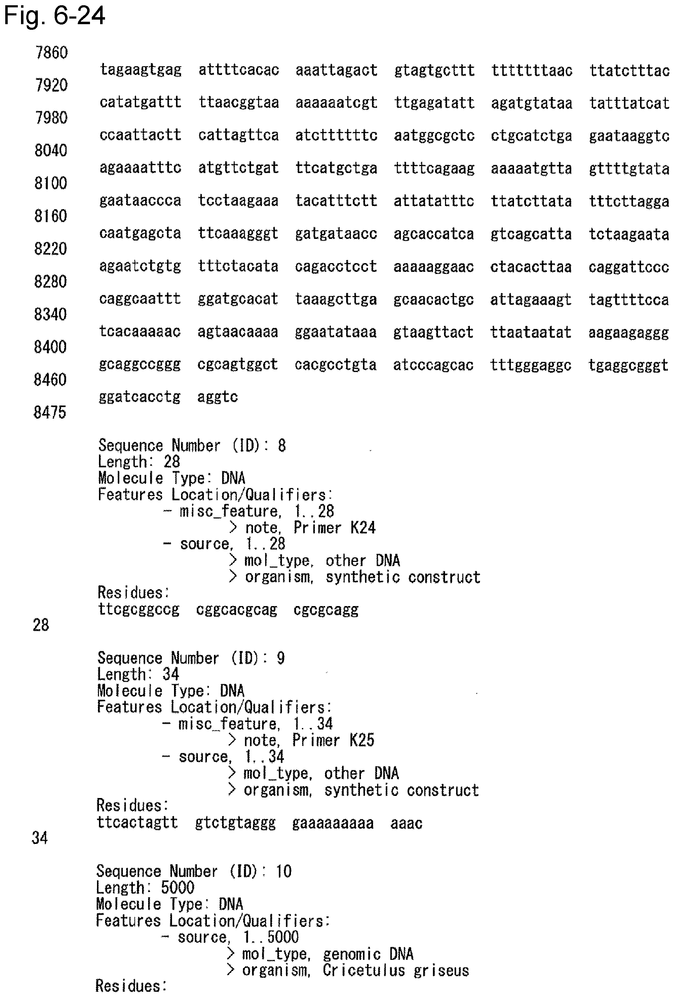

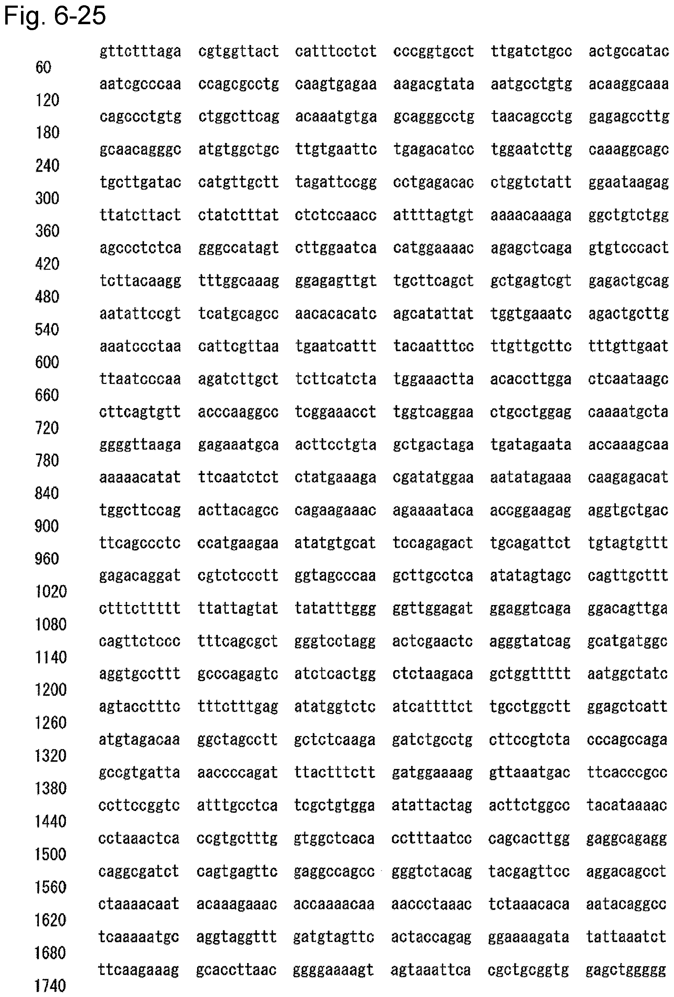

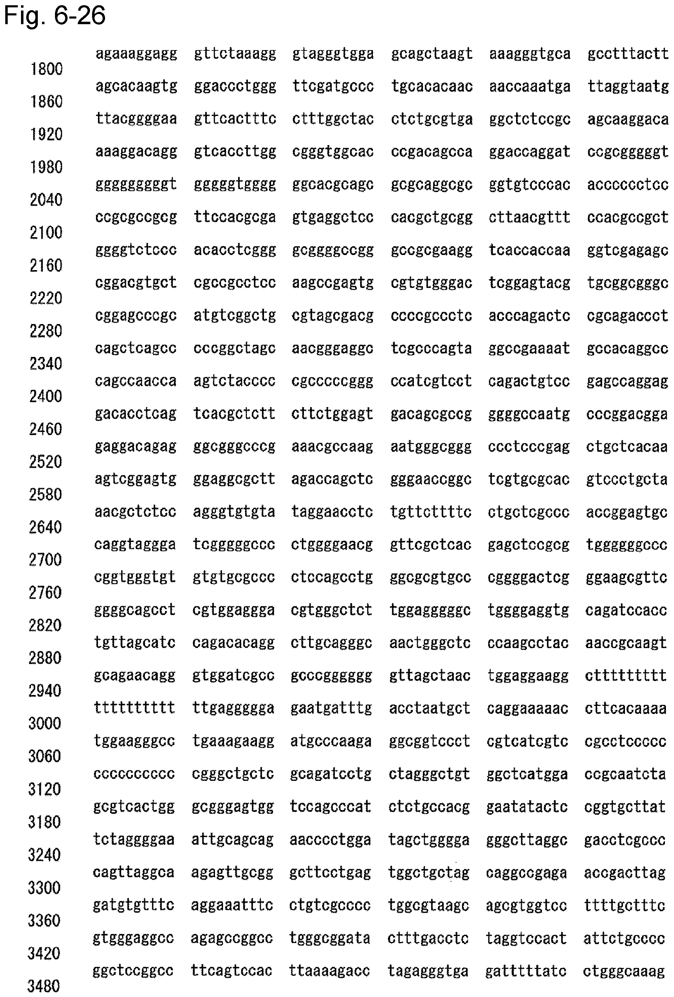

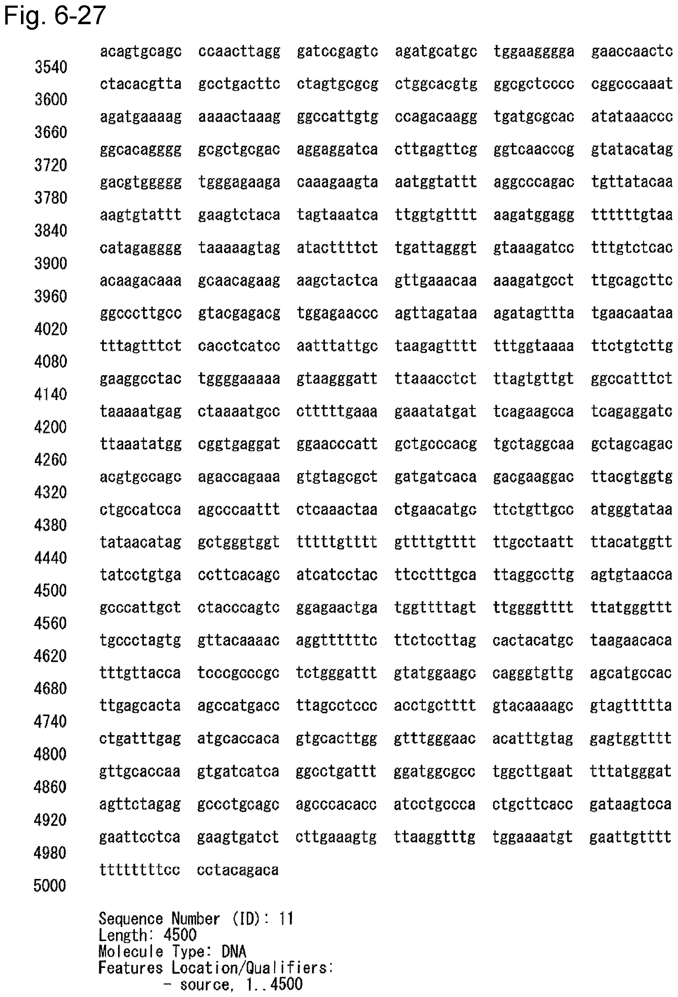

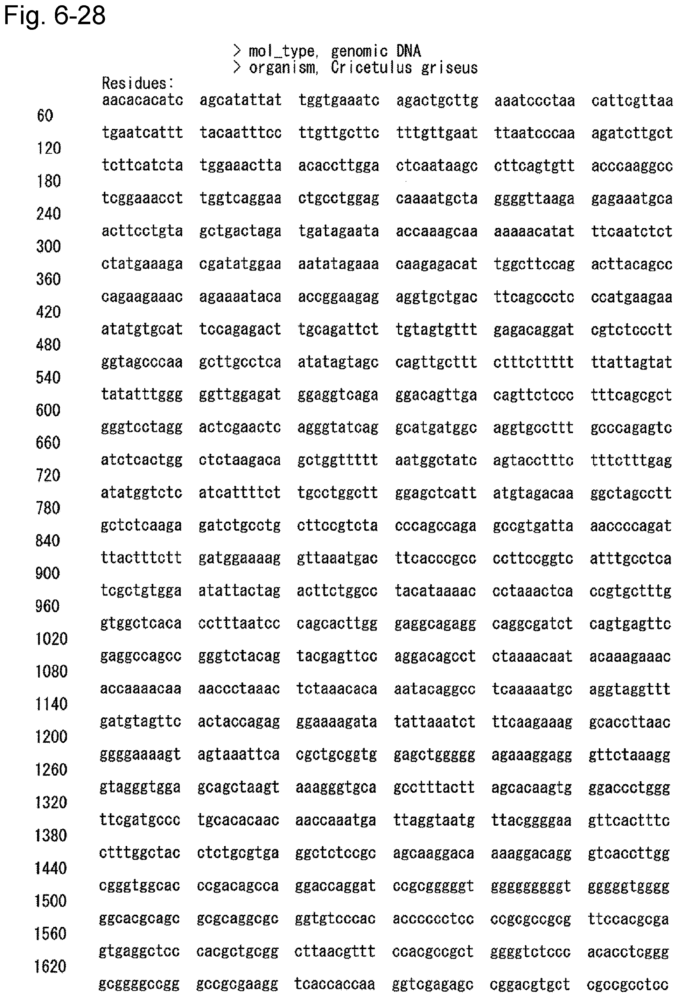

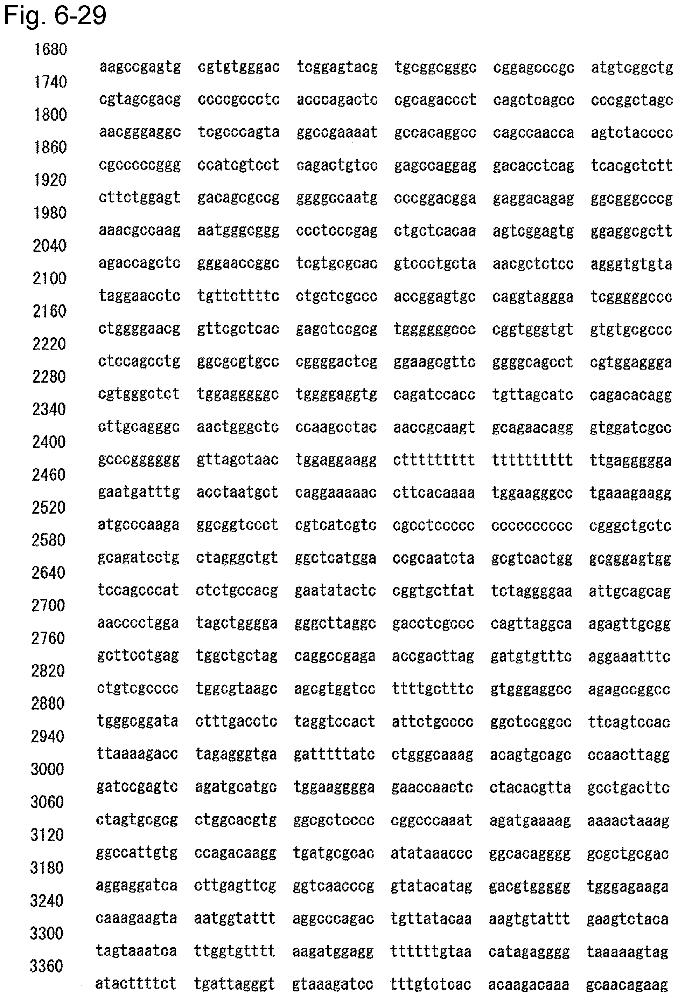

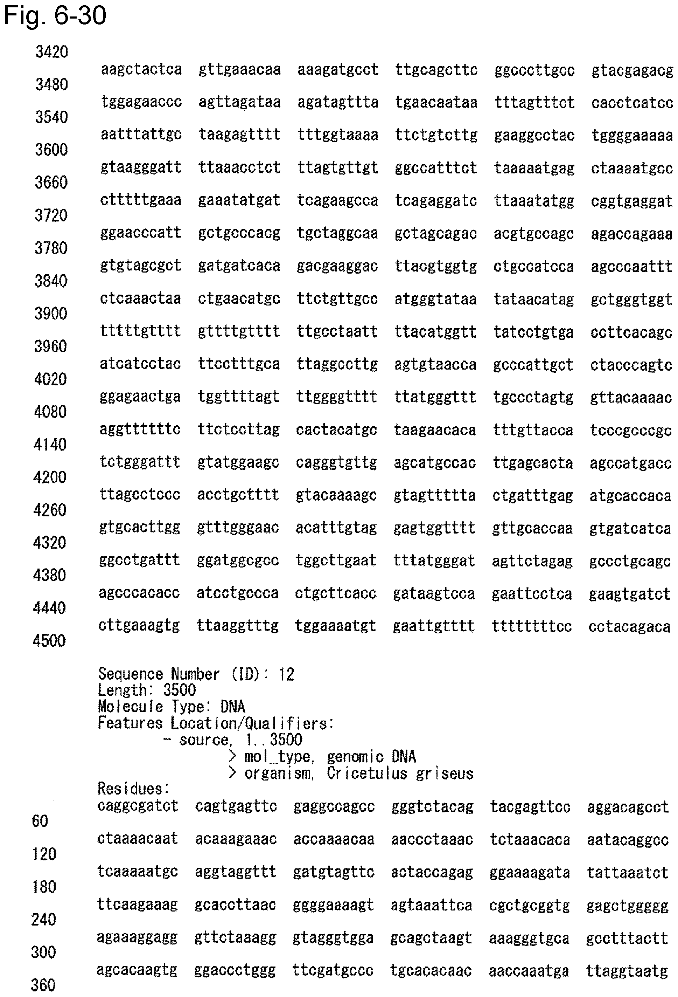

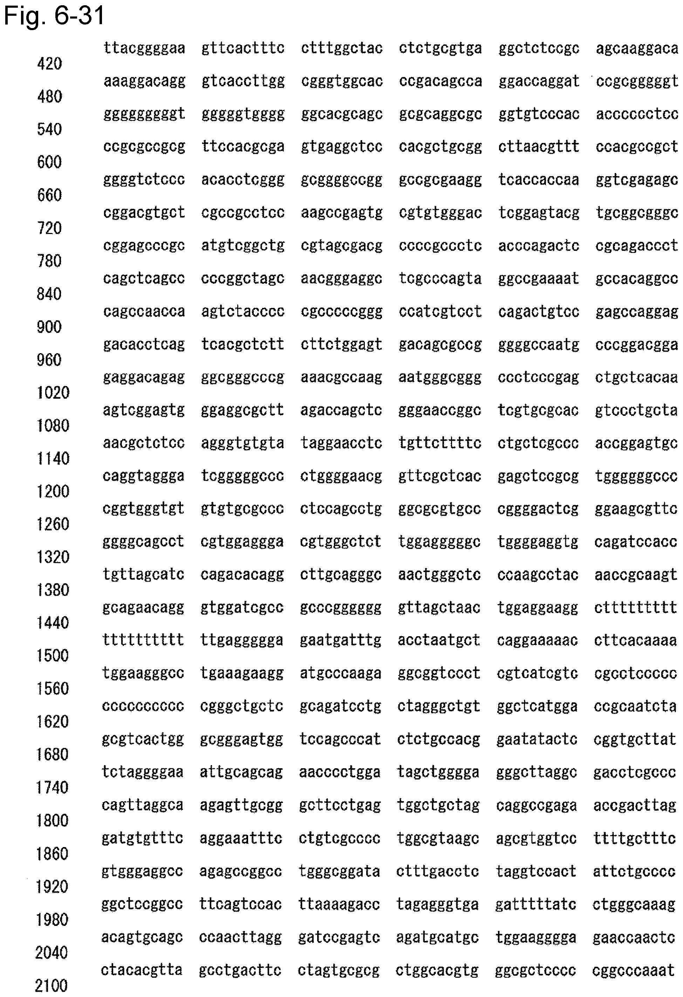

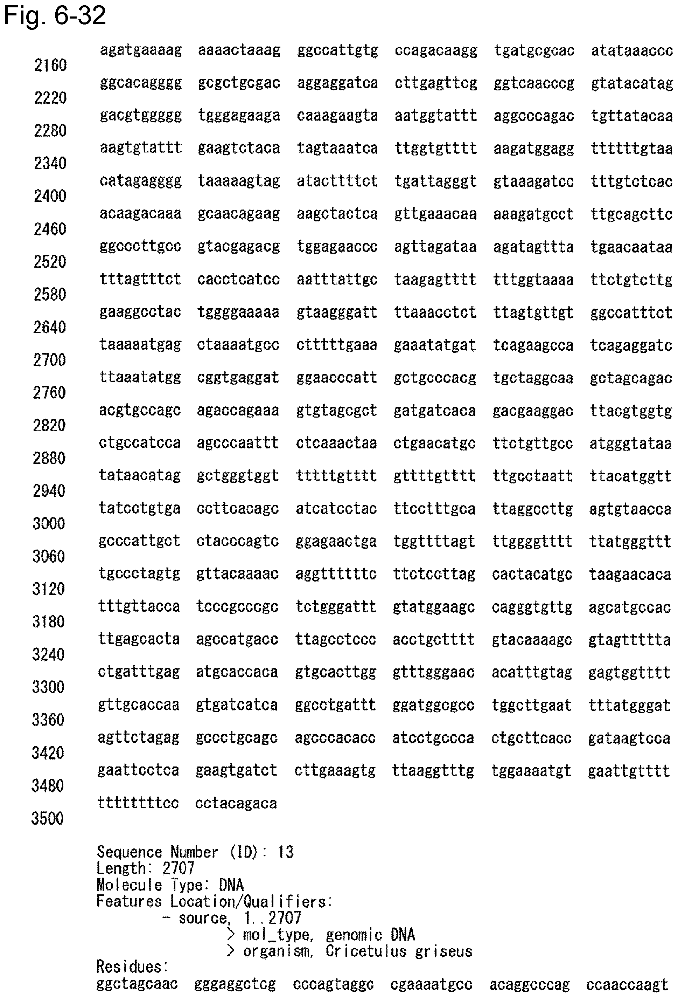

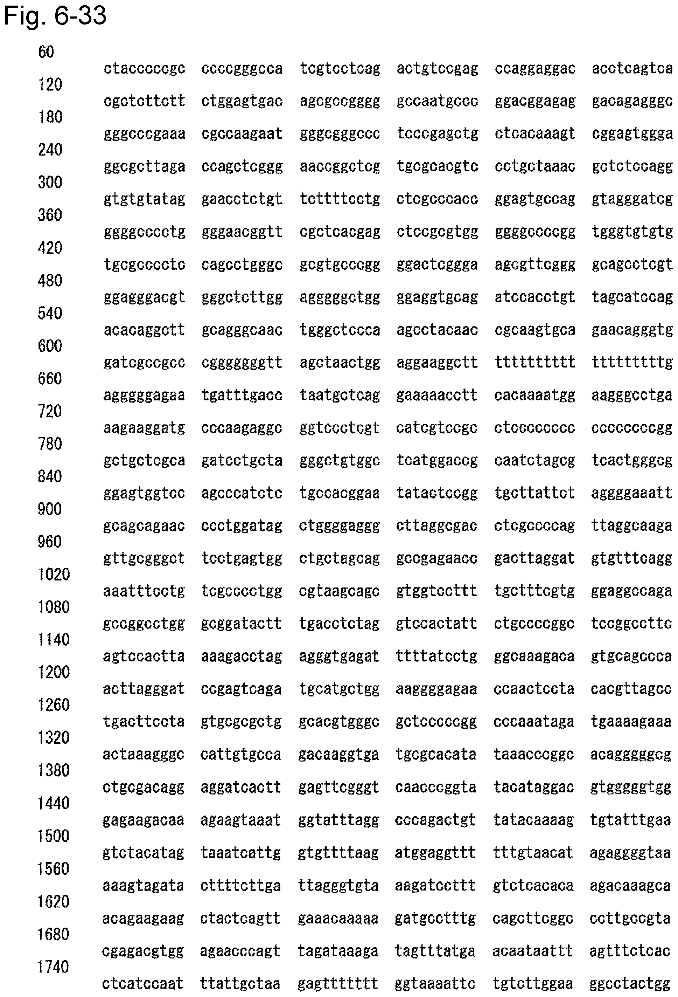

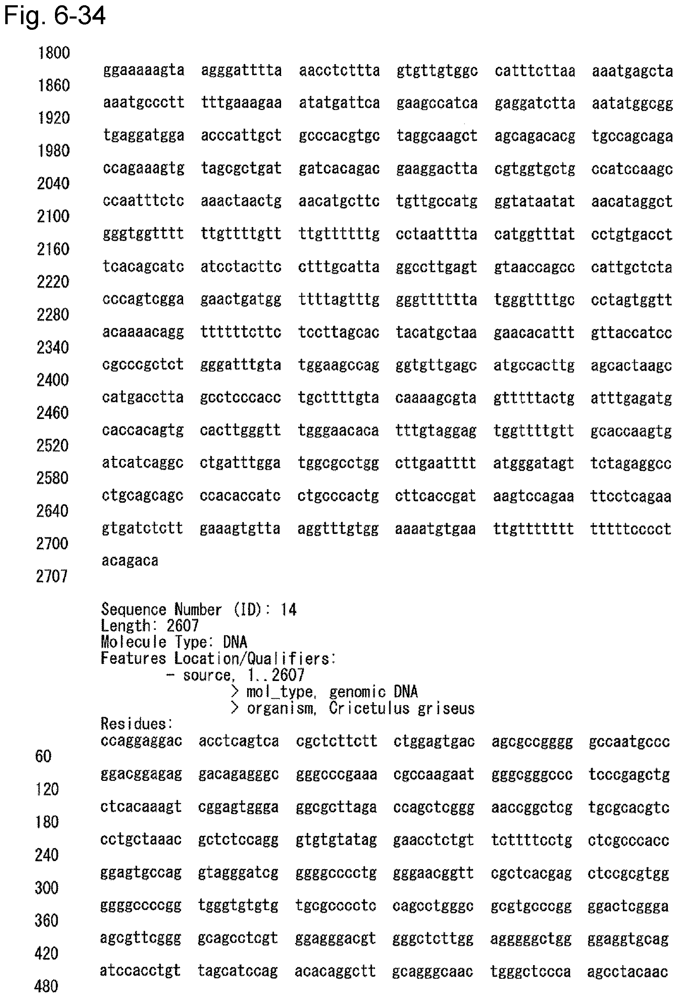

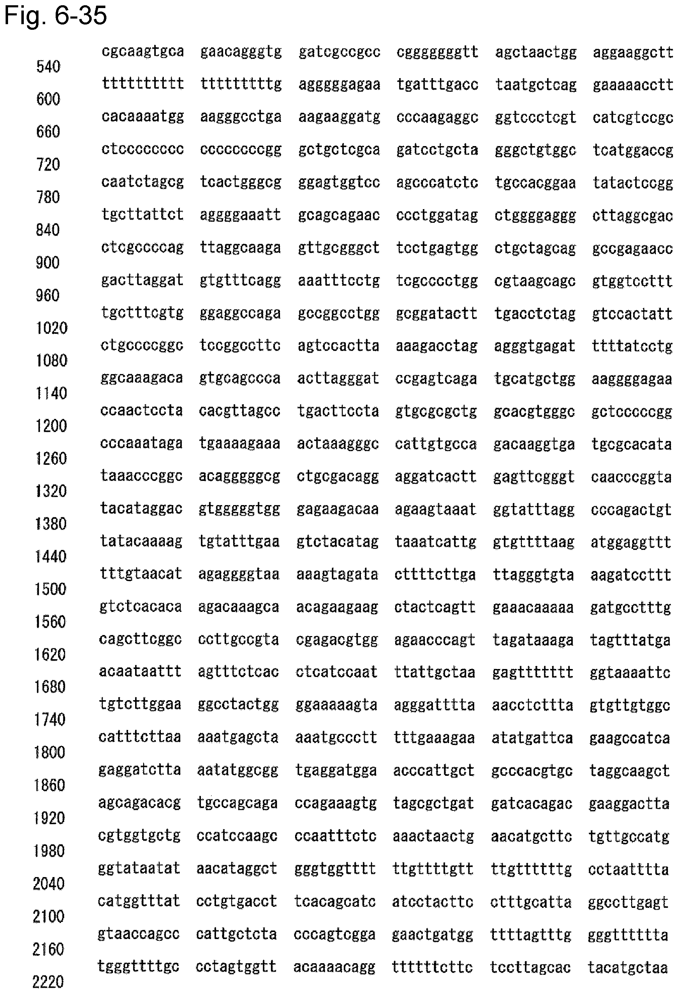

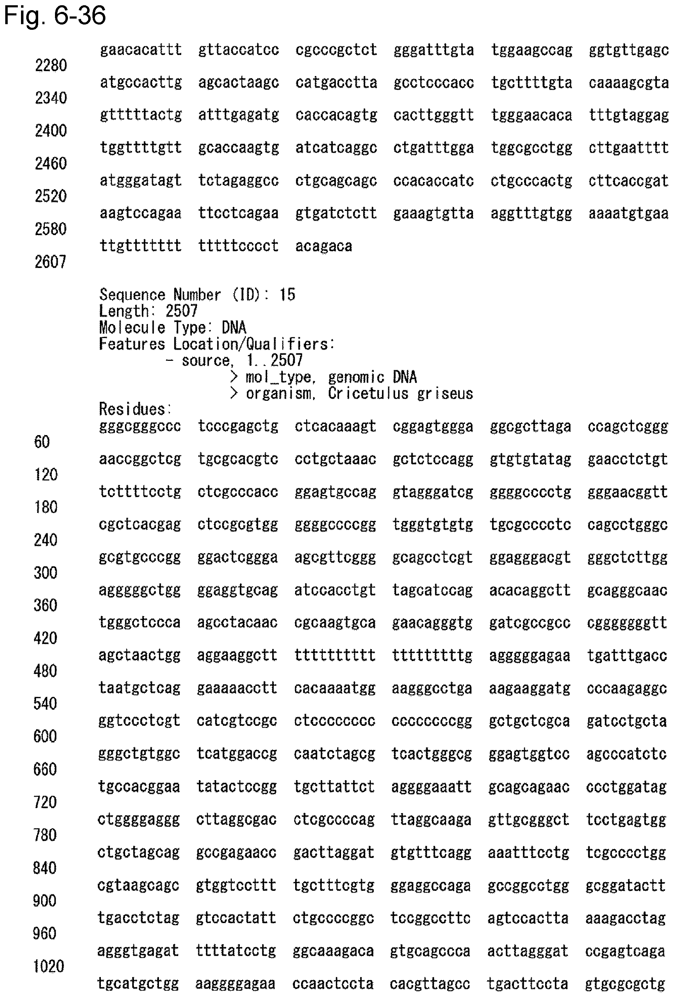

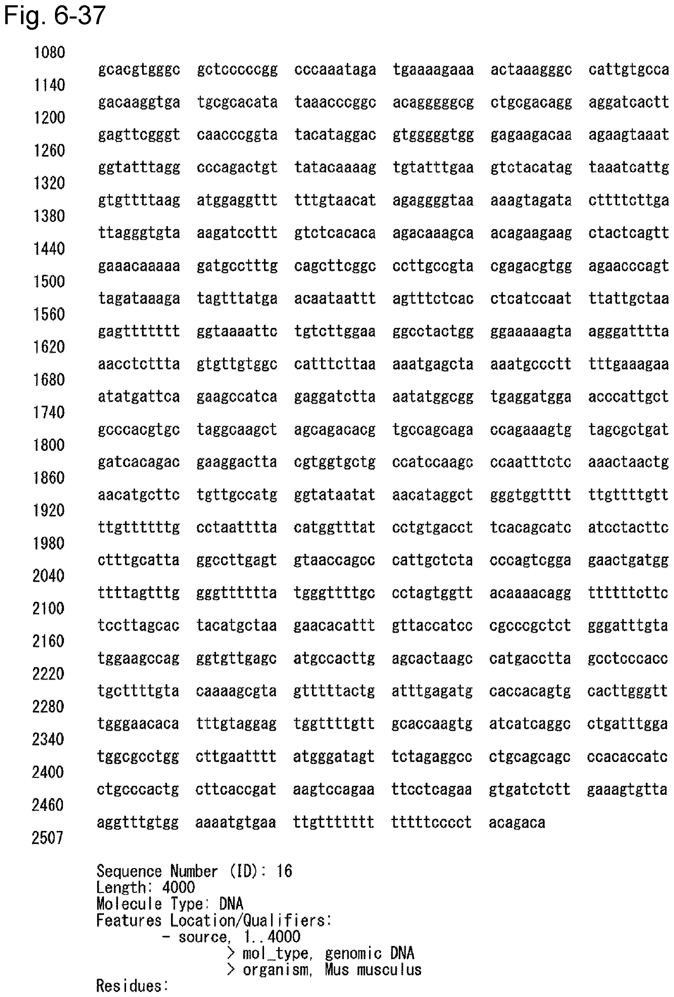

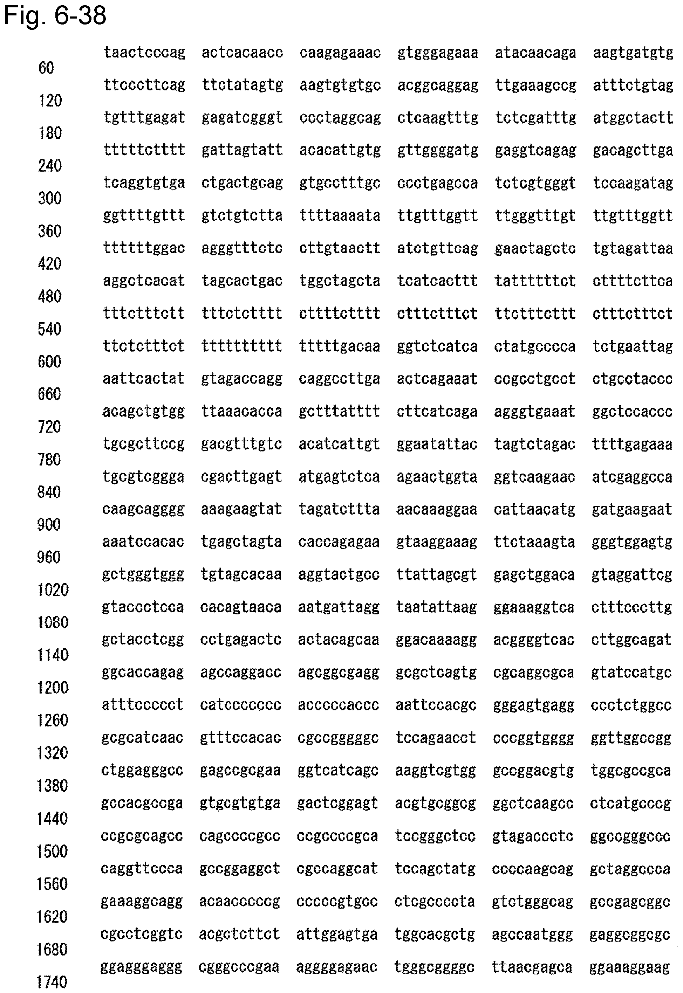

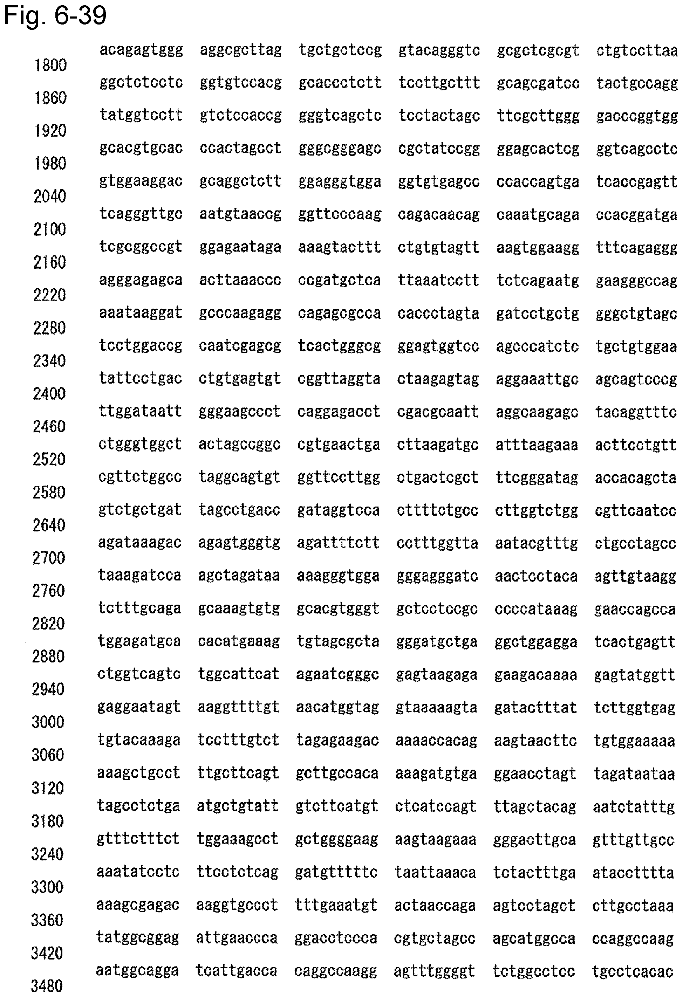

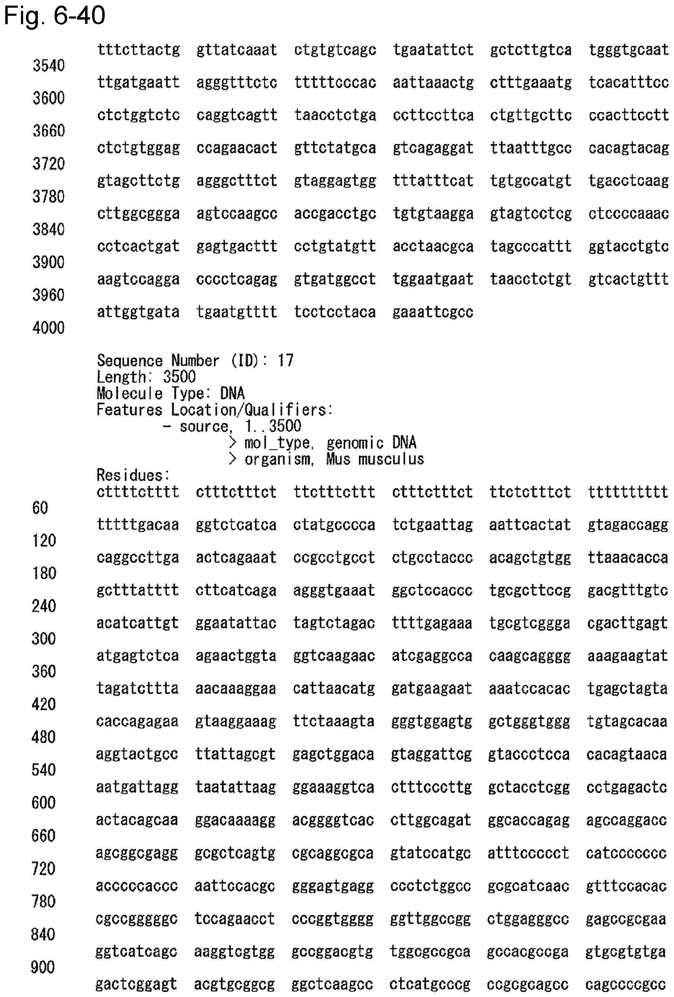

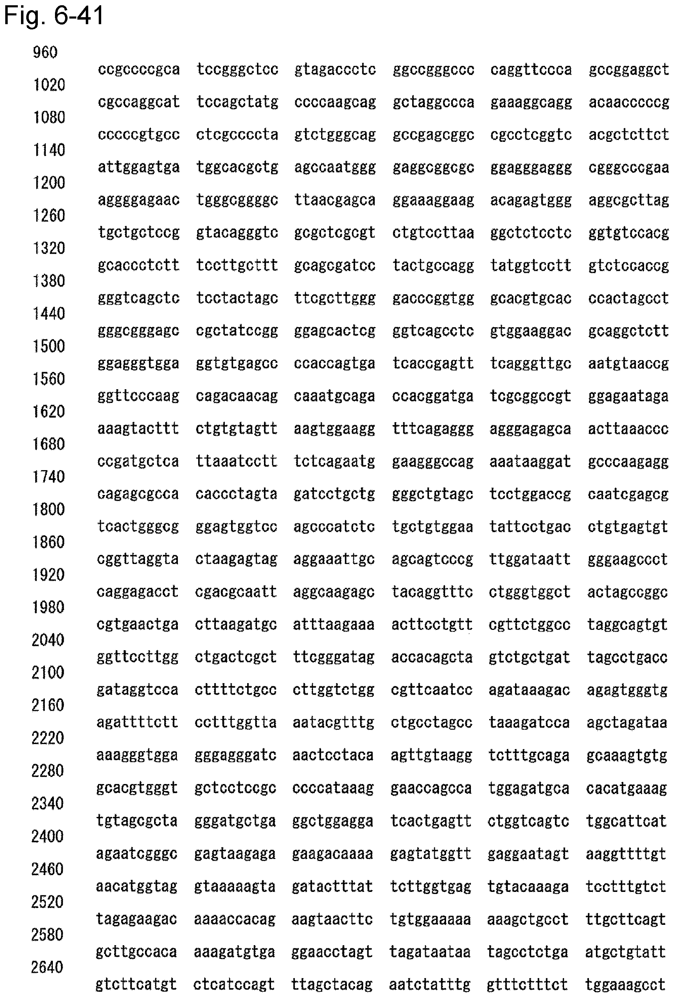

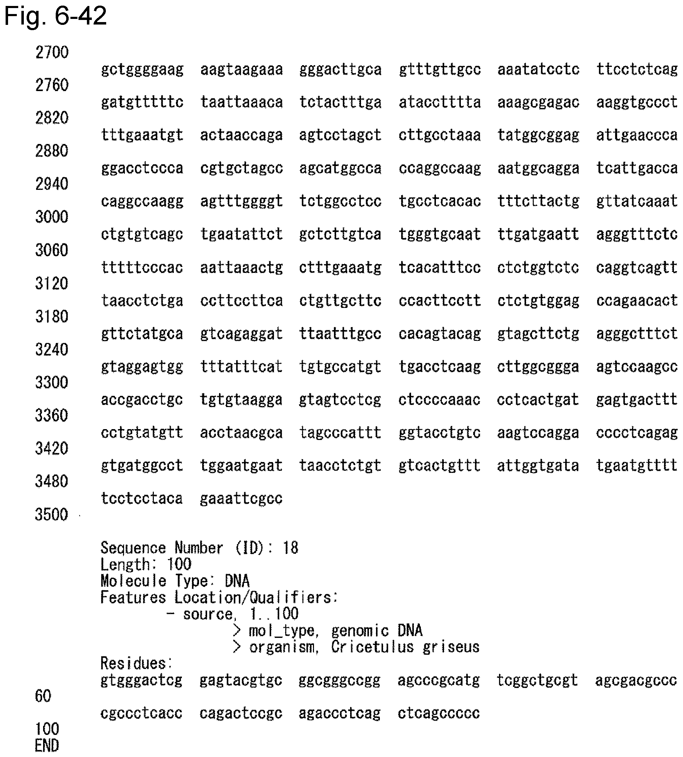

[Figure 6] Figure 6 shows a sequence listing.

### Description of Embodiments

Hereinafter, preferred forms for carrying out the present invention will be described. The embodiments described below show examples of typical embodiments of the present invention, by which the scope of the present invention should not be narrowly interpreted.

In the present description, the term "gene" means a moiety that is transcribed into mRNA and translated into a protein, and is used to include, not only DNA but also its mRNA and cDNA, and the RNA thereof.

In the present description, the term "polynucleotide" is used to have the same meaning as that of a nucleic acid, and includes DNA, RNA, a probe, an oligonucleotide, and a primer.

In the present description, the term "polypeptide" is used without being distinguished from the term "protein".

In the present description, the term "gene expression" means a phenomenon in which a gene is transcribed into mRNA, and/or a phenomenon in which the mRNA is translated into a protein.

In the present description, the term "foreign gene" means a gene that is artificially introduced into host cells.

In the present description, the term "foreign protein" means a protein encoded by the foreign gene.

In the present description, the term "gene expression unit" means a polynucleotide having at least a promoter region, a foreign gene, and a transcriptional terminator region (polyA addition signal) in the reading frame direction of transcription.

In the present description, the term "promoter" means a region to which a transcription factor involved in the start of transcription of DNA into RNA binds. In the present description, the term "promoter region" is also used. Examples of the promoter can include a polynucleotide from a nucleotide approximately 3 kbp upstream of a start codon to a nucleotide immediately upstream of a nucleotide sequence corresponding to the start codon. The promoter may contain 5'UTR and an intron.

In the present description, the term "promoter activity" refers to activity by which transcription starts through the binding of a transcription factor to the promoter to perform the production of a protein encoded by the gene. Promoter activity can be examined by using, as an indicator, the activity of a protein encoded by a reporter gene, such as firefly luciferase.

In the present description, the term "having promoter activity" means the confirmation of antibody expression under conditions similar to those used to evaluate promoter activity indicated by the amount of antibody expressed in the fed-batch culture described in (Example 2), or the confirmation of activity of firefly luciferase under conditions similar to those described in (Example 3).

In the present description, the term "DNA element" means a polynucleotide having foreign gene expression-enhancing activity when located in proximity to a gene expression unit or located in a foreign gene expression vector comprising the gene expression unit.

In the present description, the term "antigen-binding fragment of the antibody" means a partial fragment of the antibody having binding activity to the antigen. Examples thereof include Fab and F(ab')₂, though the antigen-binding fragment is not limited to these molecules as long as it has antigen-binding ability.

In the present description, the term "sequence identity" refers to the relationship between sequences as to two or more nucleotide sequences or amino acid sequences and is determined by comparison of the sequences, as known in the art. The term "sequence identity" in the art means, in some cases, the degree to which the sequence of a nucleic acid molecule or a polypeptide matches when comparing between two or more nucleotide sequences or between two or more amino acid sequences. The term "sequence identity" can be evaluated by calculating percentage identity between a smaller sequence of two or more sequences and gap alignment (if present) addressed by a particular mathematical model or computer program (i.e., "algorithm"). Specifically, the identity can be evaluated using software such as ClustalW2 provided by European Molecular Biology Laboratory-European Bioinformatics Institute (EMBL-EBI), though the evaluation method is not limited thereto as long as the method is used by a person skilled in the art.

In the present description, the phrase "to hybridize under stringent conditions" refers to hybridization under conditions in which a so-called specific hybrid is formed whereas a non-specific hybrid is not formed. Examples thereof can include conditions under which a complementary strand of a nucleic acid consisting of a nucleotide sequence having 80% or higher, preferably 90% or higher, more preferably 95% or higher, most preferably 99% or higher sequence identity to a nucleic acid hybridizes whereas a complementary strand of a nucleic acid consisting of a nucleotide sequence having lower sequence identity does not hybridize. More specifically, the phrase is used to mean that hybridization is carried out in the commercially available hybridization solution ExpressHyb Hybridization Solution (manufactured by Clontech) at 68°C, or that hybridization is carried out under conditions using a DNA-immobilized filter in the presence of 0.7 to 1.0 M NaCl at 68°C, and the resultant is then washed at 68°C with a 0.1 × to 2 × SSC solution (wherein 1 × SSC consists of 150 mM NaCl and 15 mM sodium citrate), or conditions equivalent thereto.

In the present description, the term "approximately" refers to a value that varies by up to 10%, 8%, 6%, 5%, 4%, 3%, 2% or 1% in either a plus or minus direction with respect to a reference value. Preferably, the term "approximately" refers to a range of 10%, 5% or 1% in either a plus or minus direction with respect to a reference value.

### 1. Promoter for use in enhancement of foreign gene expression

The promoter for use in the enhancement of foreign gene expression according to the present invention (hereinafter, also referred to as the "promoter of the present invention") is a promoter of an α-enolase (alpha-enolase) (hereinafter, referred to "Enol") gene.

The origin of the Enol gene promoter is not particularly limited and may be of mammalian origin. Examples thereof can include Chinese hamster, human, and mouse derived Enol gene promoters. The promoter of the present invention is preferably a rodent-derived Enol gene promoter, more preferably a Chinese hamster-, or mouse-derived Enol gene promoter. The promoter of the present invention is still more preferably a Chinese hamster-derived Enol gene promoter.

Examples of the Chinese hamster-derived Enol gene promoter include a sequence from a nucleotide that is, for example, 2.0 kbp, 2.5 kbp, 2.6 kbp, 2.7 kbp, 2.8 kbp, 3.0 kbp, 3.5 kbp, 4.0 kbp, 4.5 kbp, 5.0 kbp, 5.5 kbp or 6.0 kbp upstream of the start codon of the Enol gene, to a nucleotide immediately upstream of a nucleotide sequence corresponding to the start codon.

More specifically, examples of the Chinese hamster-derived Enol gene promoter include the polynucleotides of SEQ ID NOs: 1, 3, 4, 10, 11, 12, 13 and 14.

The nucleotide sequence of SEQ ID NO: 1 is a sequence from a nucleotide approximately 3.0 kbp upstream of the start codon of the Chinese hamster-derived Enol gene to the nucleotide immediately upstream of the nucleotide sequence corresponding to the start codon.

The nucleotide sequence of SEQ ID NO: 3 is a sequence from a nucleotide approximately 2.8 kbp upstream of the start codon of the Chinese hamster-derived Enol gene to the nucleotide immediately upstream of the nucleotide sequence corresponding to the start codon.

The nucleotide sequence of SEQ ID NO: 4 is a sequence from a nucleotide approximately 4.0 kbp upstream of the start codon of the Chinese hamster-derived Enol gene to the nucleotide immediately upstream of the nucleotide sequence corresponding to the start codon.

The nucleotide sequence of SEQ ID NO: 10 is a sequence from a nucleotide approximately 5.0 kbp upstream of the start codon of the Chinese hamster-derived Enol gene to the nucleotide immediately upstream of the nucleotide sequence corresponding to the start codon.

The nucleotide sequence of SEQ ID NO: 11 is a sequence from a nucleotide approximately 4.5 kbp upstream of the start codon of the Chinese hamster-derived Enol gene to the nucleotide immediately upstream of the nucleotide sequence corresponding to the start codon.

The nucleotide sequence of SEQ ID NO: 12 is a sequence from a nucleotide approximately 3.5 kbp upstream of the start codon of the Chinese hamster-derived Enol gene to the nucleotide immediately upstream of the nucleotide sequence corresponding to the start codon.

The nucleotide sequence of SEQ ID NO: 13 is a sequence from a nucleotide approximately 2.7 kbp upstream of the start codon of the Chinese hamster-derived Enol gene to the nucleotide immediately upstream of the nucleotide sequence corresponding to the start codon.

The nucleotide sequence of SEQ ID NO: 14 is a sequence from a nucleotide approximately 2.6 kbp upstream of the start codon of the Chinese hamster-derived Enol gene to the nucleotide immediately upstream of the nucleotide sequence corresponding to the start codon.

The Enol gene promoter can be a region having a length such that it extends to approximately 2.8 kbp or more upstream of the start codon sequence from a nucleotide immediately upstream of the nucleotide sequence corresponding to the start codon sequence because the promoter has excellent transcriptional activity. Therefore, from the viewpoint of excellent promoter activity, the Chinese hamster-derived Enol gene promoter is preferably the polynucleotide of SEQ ID NO: 1, 3, 4, 10, 11 or 12. The polynucleotide that can be selected as the Chinese hamster-derived Enol gene promoter is more preferably the polynucleotide of SEQ ID NO: 1, 3, 4 or 12, still more preferably the polynucleotide of SEQ ID NO: 1 or 3.

Examples of the mouse-derived Enol gene promoter include a sequence from a nucleotide that is, for example, 2.0 kbp, 2.5 kbp, 3.0 kbp, 3.5 kbp, 4.0 kbp, 4.5 kbp, 5.0 kbp, 5.5 kbp or 6.0 kbp upstream of the start codon of the Enol gene, to a nucleotide immediately upstream of the nucleotide sequence corresponding to the start codon.

More specifically, examples of the mouse-derived Enol gene promoter include the polynucleotides of SEQ ID NOs: 2, 17 and 16.

The nucleotide sequence of SEQ ID NO: 2 is a sequence from a nucleotide approximately 3.0 kbp upstream of the start codon of the mouse-derived Enol gene to the nucleotide immediately upstream of the nucleotide sequence corresponding to the start codon.

The nucleotide sequence of SEQ ID NO: 17 is a sequence from a nucleotide approximately 3.5 kbp upstream of the start codon of the mouse-derived Enol gene to the nucleotide immediately upstream of the nucleotide sequence corresponding to the start codon.

The nucleotide sequence of SEQ ID NO: 16 is a sequence from a nucleotide approximately 4.0 kbp upstream of the start codon of the mouse-derived Enol gene to the nucleotide immediately upstream of the nucleotide sequence corresponding to the start codon.

The promoter of the present invention may be a polynucleotide comprising a nucleotide sequence having 80% or higher, preferably 85% or higher, 86% or higher, 87% or higher, 88% or higher, 89% or higher, more preferably 90% or higher, 91% or higher, 92% or higher, 93% or higher, 94% or higher, still more preferably 95% or higher, 96% or higher, 97% or higher, 98% or higher, most preferably 99% or higher, or 100% sequence identity to the nucleotide sequence of any one of SEQ ID NOs: 1, 2, 17, 3, 4, 10, 11, 12, 13, 14 and 16, or a partial sequence of the nucleotide sequence, and having promoter activity.

The partial sequence may be a fragment whose length is 80% or more, preferably 85% or more, 86% or more, 87% or more, 88% or more, 89% or more, more preferably 90% or more, 91% or more, 92% or more, 93% or more, 94% or more, still more preferably 95% or more, 96% or more, 97% or more, 98% or more, most preferably 99% or more, or 100% of the full length of the nucleotide sequence of any one of SEQ ID NOs: 1, 2, 17, 3, 4, 10, 11, 12, 13, 14 and 16. The partial sequence may be a fragment on the 5' side (upstream) or the 3' side (downstream) of the full-length sequence, or may be a fragment obtained by connecting a plurality of fragments of the full-length sequence.

The promoter of the present invention may be a polynucleotide that hybridizes under stringent conditions to a polynucleotide consisting of a nucleotide sequence complementary to a polynucleotide consisting of the nucleotide sequence of any one of SEQ ID NOs: 1, 2, 17, 3, 4, 10, 11, 12, 13, 14 and 16, and has promoter activity.

The promoter of the present invention preferably comprises the nucleotide sequence of SEQ ID NO: 18, or a nucleotide sequence having 85% or higher sequence identity, more preferably 90% or higher, 91% or higher, 92% or higher, 93% or higher, or 94% or higher sequence identity, still more preferably 95% or higher, 96% or higher, 97% or higher, or 98% or higher sequence identity, most preferably 99% or higher, or 100% sequence identity to the nucleotide sequence.

In particular, the Chinese hamster-derived Enol promoter preferably comprises the nucleotide sequence of SEQ ID NO: 18, or a nucleotide sequence having 85% or higher sequence identity, more preferably 90% or higher, 91% or higher, 92% or higher, 93% or higher, or 94% or higher sequence identity, still more preferably 95% or higher, 96% or higher, 97% or higher, or 98% or higher sequence identity, most preferably 99% or higher, or 100% sequence identity to the nucleotide sequence.

The length of the promoter of the present invention is not particularly limited as long as it is 1 kbp or more and 10 kbp or less, and the length of the promoter is preferably 2 kbp or more and 6 kbp or less, more preferably 2.6 kbp or more and 5 kbp or less, still more preferably 2.7 kbp or more and 4.5 kbp or less, particularly preferably 2.8 kbp or more and 4 kbp or less, and can be typically approximately 3 kbp. In the case of a promoter length ensuring sufficient promoter performance, it becomes easier to exhibit an effect in terms of transfection efficiency as the promoter length decreases.

The promoter of the present invention may be a modified polynucleotide consisting of a nucleotide sequence comprising a deletion, substitution, and/or addition of one or more, preferably 1 to 300, more preferably 1 to 200, still more preferably 1 to 100, particularly preferably 1 to 30 nucleotides in the nucleotide sequence of any one of SEQ ID NOs: 1, 2, 17, 3, 4, 10, 11, 12, 13, 14 and 16.

The introduction of a modification (deletion, substitution, and/or addition) into the nucleotide sequence can be performed by an approach known in the art such as the Kunkel method or the Gapped-Duplex method, or a method equivalent thereto. For example, a kit for mutation introduction which exploits site-directed mutagenesis (e.g., Mutant-K (manufactured by Takara Bio Inc.) or Mutant-G (manufactured by Takara Bio Inc.), or an LA PCR *in vitro* Mutagenesis series kit from Takara Bio Inc.) can be utilized. Such a modified polynucleotide can also be used as the promoter of the present invention.

The foreign gene expression-enhancing activity possessed by the promoter of the present invention can be examined by using, as an indicator, the activity of a protein encoded by a reporter gene, such as firefly luciferase, or the amount of an antibody produced in fed-batch culture.

When the activity of a protein encoded by a reporter gene of a vector comprising the promoter of the present invention (e.g., the amount of luminescence of firefly luciferase) is 1 or more times, preferably 20 or more times, more preferably 30 or more times, still more preferably 40 or more times, even more preferably 45 or more times, particularly preferably 50 or more times higher compared with the activity of a protein encoded by a reporter gene of a control vector (e.g., the amount of luminescence of Renilla luciferase), it can be determined that this promoter has foreign gene expression-enhancing activity.

When the amount of the antibody produced in fed-batch culture is equivalent or higher, preferably 1.2 or more times, more preferably 1.3 or more times, still more preferably 1.5 or more times, particularly preferably 1.8 or more times, 1.9 or more times, or 2.0 or more times by use of the promoter of the present invention compared with use of a human EF-1α promoter, it can be determined that this promoter has foreign gene expression-enhancing activity. Even in the case of an enhancement of approximately 1.2-fold or more, a reduction of cell culture scale, culture time and the number of purification steps is expected. As a result, an improvement in yield and a reduction in culture cost are attained. The improved yield permits stable supply of a foreign protein as a medicament. Also, the reduced culture cost leads to a reduction in the prime cost of a foreign protein as a medicament.

### 2. Foreign gene expression unit

The foreign gene expression unit of the present invention (hereinafter, also referred to as the "gene expression unit of the present invention") has at least the promoter of the present invention described in the preceding section 1., a foreign gene, and a transcriptional terminator region (polyA addition signal) in the reading frame direction of transcription.

The polyA addition sequence can be any sequence having the activity of terminating transcription from the promoter, and may be derived from a gene which is the same as or different from the gene of the promoter.

### 3. DNA element for use in enhancing foreign gene expression

Combined use of the gene expression unit of the present invention described in the preceding section 2. with a DNA element can further enhance the expression of a foreign gene. The DNA element for combined use can be obtained by interaction with acetylated histone H3 as an indicator. In general, the acetylation of histone (H3, H4) is reportedly involved in the activation of transcription on the basis of two main hypotheses: that conformational change of the nucleosome is involved such that histone tail acetylation neutralizes the charge thereof to loosen the binding between the DNA and the histone (Mellor J. (2006), Dynamic nucleosomes and gene transcription, Trends Genet. 22 (6): 320-329); and that the acetylation is involved in the recruitment of various transcription factors (Nakatani Y. (2001), Histone acetylases - versatile players, Genes Cells. 6 (2): 79-86). Both of the hypotheses strongly suggest that the acetylation of histone is involved in transcriptional activation. Thus, chromatin immunoprecipitation (ChIP) using an anti-acetylated histone H3 antibody is capable of enriching a sample for a DNA element that interacts with acetylated histone H3.

Examples of the DNA element for use in the enhancing of foreign gene expression in combination with the promoter of the present invention can include A2, A7, and A18. The DNA element is preferably A7.

A2 is positioned at a site from 80966429 to 80974878 of human chromosome 15 and is an 8450 bp polynucleotide having an AT content of 62.2%. The nucleotide sequence of A2 is shown in SEQ ID NO: 5 in the sequence listing.

A7 is positioned at a site from 88992123 to 89000542 of human chromosome 11 and is an 8420 bp polynucleotide having an AT content of 64.52%. The nucleotide sequence of A7 is shown in SEQ ID NO: 6 in the sequence listing.

A18 is positioned at a site from 111275976 to 111284450 of human chromosome 4 and is an 8475 bp polynucleotide having an AT content of 62.54%. The nucleotide sequence of A18 is shown in SEQ ID NO: 7 in the sequence listing.

The foreign gene expression-enhancing activity possessed by the DNA element for combined use with the promoter of the present invention can be examined by using, as an indicator, the activity of a protein encoded by a reporter gene, such as the secreted alkaline phosphatase (SEAP).

For combined use with the promoter of the present invention, any one of the DNA elements described above may be used alone, or two or more copies of one DNA element may be used. Alternatively, two or more DNA elements may be used in combination.

The DNA element used in the present invention may consist of a nucleotide sequence having 80% or higher, preferably 85% or higher, 86% or higher, 87% or higher, 88% or higher, or 89% or higher, more preferably 90% or higher, 91% or higher, 92% or higher, 93% or higher, or 94% or higher, still more preferably 95% or higher, 96% or higher, 97% or higher, 98% or higher, most preferably 99% or higher, or 100% identity to the nucleotide sequence of any one of SEQ ID NOs: 5 to 7, and having foreign gene expression-enhancing activity.

The DNA element used in the present invention may be a modified polynucleotide consisting of a nucleotide sequence comprising a deletion, substitution, and/or addition of one or more, preferably 1 to 1000 or 1 to 500, more preferably 1 to 100 or 1 to 50, still more preferably 1 to 10 or 1 to 5, particularly preferably 4, 3, 2 or 1 nucleotides in the nucleotide sequence of any one of SEQ ID NOs: 5 to 7, and having foreign gene expression-enhancing activity.

The introduction of a modification (deletion, substitution, and/or addition) into the polynucleotide can be performed by an approach known in the art such as the Kunkel method or the Gapped-Duplex method, or a method equivalent thereto. For example, a kit for mutation introduction (e.g., Mutant-K (manufactured by Takara Bio Inc.) or Mutant-G (manufactured by Takara Bio Inc.), or an LA PCR in vitro Mutagenesis series kit from Takara Bio Inc.) can be utilized which exploits site-directed mutagenesis. Such a mutant polynucleotide can also be used as the DNA element of the present invention.

As the DNA element used in the present invention, a partial sequence consisting of at least 3000 or at least 2000 consecutive nucleotides in the polynucleotide sequence shown in any one of SEQ ID NOs: 5 to 7 can be used. Such a partial sequence may be, for example, a partial sequence described in International Publication No. WO 2012/005378. Examples of the partial sequence A2 can include A2-1 to A2-17, examples of the partial sequence A7 can include A7-1 to A7-18, and examples of the partial sequence A18 can include A18-1 to A18-4.

In the present invention, any one of the partial sequences of the DNA element described above may be used alone, or two or more copies of one partial fragment may be used. Alternatively, two or more partial fragments may be used in combination. Full-length sequences and partial sequences of the DNA elements may be used in combination. In the combination, the partial element may be derived from a DNA element of a full-length sequence forming the combination, or may be derived from a DNA element of a different full-length sequence.

### 4. Obtaining the polynucleotide

In the present invention, a polynucleotide comprising a foreign gene encoding a foreign protein whose production is to be enhanced as mentioned later can be obtained by a general method given below. The polynucleotide can be isolated, for example, by screening a cDNA library derived from cells or tissues expressing the foreign gene, using a DNA probe synthesized on the basis of a gene fragment. mRNA can be prepared by an approach usually used in the art. For example, the cells or the tissues are treated with a guanidine reagent, a phenol reagent, or the like to obtain total RNA. Then, poly(A)⁺ RNA (mRNA) is obtained therefrom by an affinity column method using an oligo(dT) cellulose column, polyU-Sepharose with Sepharose 2B as a carrier, or the like, or by a batch method. The poly(A) ⁺ RNA may be further fractionated by a sucrose density gradient centrifugation method or the like. Subsequently, single-stranded cDNA is synthesized with the obtained mRNA as a template using an oligo dT primer and reverse transcriptase. Double-stranded cDNA is synthesized from the single-stranded cDNA using DNA synthetase I, DNA ligase and RNase H, etc. The synthesized double-stranded cDNA is converted into a blunt-ended form with T4 DNA synthetase, then subjected to the linkage of an adaptor (e.g., an EcoRI adaptor), phosphorylation, etc., and incorporated into a λ phage such as λgt11 for *in vivo* packaging to prepare a cDNA library. Alternatively, the cDNA library may be prepared using a plasmid vector instead of the λ phage. Then, a clone having the DNA of interest (a positive clone) can be selected from the cDNA library.

In the case of isolating a polynucleotide comprising the promoter and a terminator region, the DNA element, or a polynucleotide comprising a foreign gene for use in protein production from genomic DNA, the genomic DNA is extracted from a cell line of an organism serving as a source, followed by polynucleotide selection, according to a general approach (Molecular Cloning (1989) and Methods in Enzymology 194 (1991)). The extraction of the genomic DNA can be performed according to, for example, the method of Cryer et al. (Methods in Cell Biology, 12, 39-44 (1975)) and the method of P. Philippsen et al. (Methods Enzymol., 194, 169-182 (1991)).

The obtaining of the polynucleotide of interest comprising the promoter, the DNA element, or a foreign gene can also be performed by, for example, PCR (PCR Technology, Henry A. Erlich, Atockton press (1989)). The amplification of the polynucleotide by PCR employs a 20 to 30 mer synthetic single-stranded DNA as a primer and genomic DNA as a template. The amplified gene is used after its polynucleotide sequence is confirmed. A genomic DNA library such as a bacterial artificial chromosome (BAC) library may be used as a template for PCR.

On the other hand, a polynucleotide comprising a foreign gene having an unknown sequence can be obtained by (a) preparing a gene library according to a common method, (b) selecting the desired polynucleotide from the prepared gene library, and amplifying the polynucleotide. The gene library can be prepared by partially digesting chromosomal DNA obtained by a common method from a cell line of an organism serving as a source, with an appropriate restriction enzyme to prepare fragments, ligating the obtained fragments into an appropriate vector, and introducing the vector into an appropriate host. Alternatively, the gene library may be prepared by extracting mRNA from the cells, synthesizing cDNA therefrom, then ligating the cDNA into an appropriate vector, and introducing the vector into an appropriate host. In this respect, a plasmid known as a well-known vector for gene library preparation can be used as the vector, and a phage vector or a cosmid, etc. can also be widely used. The host to be transfected or transduced can be used according to the type of vector. The polynucleotide comprising a foreign gene is selected from the gene library by colony hybridization, plaque hybridization, or the like using a labeled probe comprising a sequence unique to the foreign gene.

The polynucleotide comprising a foreign gene can also be synthesized entirely chemically. The gene can be synthesized by, for example, a method of preparing two pairs of complementary oligonucleotides and annealing these, a method of ligating several annealed DNAs using DNA ligase, or a method of preparing several partially complementary oligonucleotides and filling the gaps therein by PCR.

The polynucleotide sequence can be determined by a usual method, for example, the dideoxy method (Sanger et al., Proc. Natl. Acad. Sci., USA, 74, 5463-5467 (1977)). Alternatively, the polynucleotide sequence may be readily determined using a commercially available sequencing kit or the like.

### 5. Foreign gene expression vector

A vector comprising the foreign gene expression unit described in the preceding section 2. comprising the promoter described in the preceding section 1. is provided as a foreign gene expression vector of the present invention. The foreign gene expression vector of the present invention may comprise one of the DNA elements described in the preceding section 3., two or more copies of one DNA element, or a combination of two or more DNA elements. When a foreign gene is expressed in host cells using the foreign gene expression vector, the DNA element may be located immediately preceding or immediately following the gene expression unit, or may be located at a position distant from the gene expression unit. Alternatively, one foreign gene expression vector comprising a plurality of DNA elements may be used. The orientation of the DNA element may be in either the forward direction or the reverse direction with respect to the gene expression unit.

Examples of the foreign gene can include, but are not particularly limited to: reporter genes such as the secreted alkaline phosphatase (SEAP) gene, the green fluorescence protein (GFP) gene, and the luciferase gene; various enzyme genes such as the α-amylase gene and the α-galactosidase gene; genes of various interferons such as interferon α and interferon γ, which are pharmaceutically useful physiologically active proteins; genes of various interleukins such as IL1 and IL2; various cytokine genes such as the erythropoietin (EPO) gene and the granulocyte colony-stimulating factor (G-CSF) gene; growth factor genes; and a gene encoding a multimeric protein, for example, a gene encoding a heteromultimer which is an antibody or an antigen-binding fragment thereof. These genes may be obtained by any approach.

The term "antigen-binding fragment of the antibody" means a partial fragment of the antibody having binding activity to the antigen. Examples thereof include Fab, F(ab')₂, Fv, scFv, a diabody, linear antibodies, and multispecific antibodies formed from antibody fragments. Also, Fab', which is a monovalent fragment of antibody variable regions obtained by the treatment of F(ab')₂ under reductive conditions is included within the antigen-binding fragment of the antibody. However, the antigen-binding fragment is not limited to these molecules as long as it has antigen-binding ability. Furthermore, these antigen-binding fragments also include not only a fragment obtained by the treatment of the full-length molecule of an antibody protein with an appropriate enzyme but also a protein produced in appropriate host cells using a genetically engineered antibody gene.

The foreign gene expression vector of the present invention can comprise a selection marker for selecting a transfectant. The transfectant can be selected using, for example, a drug resistance marker which confers resistance to a drug such as cerulenin, aureobasidin, zeocin, canavanine, cycloheximide, hygromycin, puromycin, blasticidin, tetracycline, kanamycin, ampicillin, or neomycin. Alternatively, the transfectant may be selected by using, as a marker, a gene that confers, for example, solvent resistance to ethanol or the like, osmotic pressure resistance to glycerol, a salt, or the like, or metal ion resistance to copper or the like.

The foreign gene expression vector of the present invention may be a vector that is not integrated into chromosomal DNA. In general, the foreign gene expression vector is randomly integrated into the chromosome after transfection of host cells. By contrast, use of a constituent derived from a mammalian virus such as simian virus 40 (SV40), papillomavirus (BPV, HPV), or EBV allows the foreign gene expression vector to be used as an episomal vector capable of replicating autonomously in the transfected host cells. For example, a vector having a sequence encoding a SV40-derived replication origin and a trans-acting factor SV40 large T antigen, or a vector having a sequence encoding EBV-derived oriP and EBNA-1 is widely used. The DNA element is capable of exhibiting foreign gene expression-enhancing activity, regardless of the type of vector or the presence or absence of integration into the chromosome.

### 6. Transfected cells

The transfected cells of the present invention are transfected cells comprising the foreign gene expression vector of the preceding section 5. introduced thereinto.

The host cells to be transfected are eukaryotic cells, preferably mammalian cells, more preferably human-, mouse-, rat-, hamster-, monkey-, or bovine-derived cells. Examples of the mammalian cells can include, but are not limited to, COS-1 cells, 293 cells, and CHO cells (e.g., CHO-K1, CHO-O1, CHO DG44, CHO dhfr⁻, CHO-S).

In the present invention, the method for introducing the expression vector into host cells can be any method as long as the method allows the introduced gene to be present stably in the host and to be appropriately expressed. Examples thereof can include methods generally used, for example, the calcium phosphate method (Ito et al., (1984) Agric. Biol. Chem., 48, 341), electroporation (Becker, D.M. et al. (1990), Methods. Enzymol., 194, 182-187), the spheroplast method (Creggh et al., Mol. Cell. Biol., 5, 3376 (1985)), the lithium acetate method (Itoh, H. (1983), J. Bacteriol. 153, 163-168), and lipofection.

In the transfected cells according to the present invention, a foreign gene may be transiently expressed, or stably expressed, but is preferably expressed in a stable expression system.

The term "stable expression system" refers to a method in which an expression vector is incorporated into a chromosome by the calcium phosphate method, electroporation, lipofection or the like to perform the expression. The introduced gene is maintained on the chromosome, so that the expression of the introduced gene can be maintained for a long period of several weeks or longer. Introduction of a selection marker into plasmids enables drug selection. It is possible to efficiently select cells in which the introduced gene is maintained on the chromosome.

### 7. Method for producing foreign protein

The production of a foreign protein according to the present invention can be performed by culturing the transfected cells described in the preceding section 6. by a known method, and collecting the foreign protein from the culture, followed by purification. The "culture" means any of a culture supernatant, cultured cells, and a cell homogenate. Not only a monomeric protein but also a multimeric protein may be selected as the foreign protein that can be produced using the transfected cells described in section 6. In the case of producing a heteromultimeric protein constituted by a plurality of different subunits, a plurality of genes encoding these subunits each need to be introduced into the host cells described in section 6.

The transfected cells can be cultured according to usual methods for use in the culture of the host cells.

When the transfected cells are mammalian cells, the transfected cells are cultured, for example, at 37°C under 5% or 8% CO₂ conditions for a culture time in the order of 24 to 1000 hours. The culture can be carried out by, for example, static culture, shake culture, stirring culture, batch culture under aeration, fed-batch culture, perfusion culture or continuous culture. The culture is preferably fed-batch culture. The period of the fed-batch culture of the transfected cells may be 3 days or more and 20 days or less, and is preferably 5 days or more and 18 days or less, particularly preferably 6 days or more and 15 days or less.

The expression product of the foreign protein gene from the culture (culture solution) described above can be confirmed by SDS-PAGE, Western blotting, ELISA, or the like.

### 8. Method for producing antibody protein

Examples of the heteromultimeric protein to be produced using the production method described in the preceding section 7. can include antibody proteins. The antibody protein is a tetramer protein consisting of two molecules of a heavy chain polypeptide and two molecules of a light chain polypeptide. Thus, for obtaining an antibody protein in a form that maintains antigen-binding ability, it is necessary to introduce both, heavy chain and light chain genes, into the transfected cells described in the preceding section 6. In this case, heavy chain and light chain gene expression units may be present on the same expression vector or may be present on different expression vectors. When the gene expression units are present on the same expression vector, the light chain gene expression unit and the heavy chain gene expression unit may be present in this order, and it is preferable that the DNA element, the light chain gene expression unit and the heavy chain gene expression unit be present in this order, in a gene reading direction. More preferably, the DNA element, the light chain gene expression unit and the heavy chain gene expression unit present in this order are followed by a selection marker.

Examples of the antibody to be produced according to the present invention can include antibodies prepared by immunizing laboratory animals such as rabbits, mice, and rats with the desired antigen. Further examples of the antibody to be produced according to the present invention can include chimeric antibodies and humanized antibodies originating from the antibodies described above. In addition, a human antibody obtained from a genetically engineered animal or by the phage display method is also an antibody to be produced according to the present invention.

The antibody gene for use in antibody production is not limited to an antibody gene having a particular polynucleotide sequence as long as a combination of a heavy chain polypeptide and a light chain polypeptide obtained by the transcription of the antibody genes and subsequent translation retains the activity of binding to a given antigen protein.

The antibody gene is not necessarily required to encode the full-length molecule of the antibody. A gene encoding an antigen-binding fragment of the antibody can be used. The genes encoding such an antigen-binding fragment can be obtained by genetically engineering genes encoding the full-length molecule of the antibody protein.

### 9. Methods for producing other foreign proteins

Examples of the foreign protein to be produced by the production method of the present invention can include the antibodies mentioned above as well as various human- or non-human animal-derived proteins, antigen-binding fragments thereof, and modified forms of the proteins or the fragments. Examples of such a protein and the like can include, but are not limited to: peptide hormones such as atrial natriuretic peptide (ANP), brain natriuretic peptide (BNP), C-type natriuretic peptide (CNP), vasopressin, somatostatin, growth hormone (GH), insulin, oxytocin, ghrelin, leptin, adiponectin, renin, calcitonin, osteoprotegerin, and insulin-like growth factor (IGF); cytokines such as interleukin, chemokine, interferon, tumor necrosis factor (TNFα/β as well as TNF superfamily, etc.), nerve growth factor (NGF), cell growth factor (EGF, FGF, PDGF, HGF, TGF, etc.), hematopoietic factor (CSF, G-CSF, erythropoietin, etc.), and adiponectin; receptors such as TNF receptor; enzymes such as lysozyme, protease, proteinase, and peptidase; functional fragments thereof (fragments partially or wholly retaining the biological activity of the original protein); and fusion proteins comprising these proteins.

### Examples

Hereinafter, the present invention will be specifically described with reference to Examples. However, these Examples do not limit the technical scope of the present invention by any means. Plasmids, restriction enzymes, DNA-modifying enzymes, etc. used in the Examples of the present invention are commercially available and can be used according to common methods. Operations used in DNA cloning, polynucleotide sequencing, transfection of host cells, culture of transfected cells, collection of a protein from the resulting culture, purification, etc. are also well known to a person skilled in the art or can be derived from the literature.

### (Example 1) Cloning of an Enol gene promoter region

The Enol promoter region used was a sequence from a nucleotide approximately 3.0 kbp upstream of the start codon sequence of the Enol gene to the nucleotide immediately upstream of the nucleotide sequence corresponding to the start codon sequence with reference to the sequence of the mRNA registered under XM_027398299.1 and the scaffold sequence of the Chinese hamster genome registered under NC_048595.1 in GenBank.

The Enol gene promoter region was amplified by PCR with the genomic DNA of CHO cells as a template using the primer set given below and PrimeSTAR Max DNA Polymerase (Takara Bio Inc.), and the PCR product was purified using a QIAquick PCR Purification kit (Qiagen). The nucleotide sequence of the cloned Chinese hamster Enol gene promoter region is shown in SEQ ID NO: 1 in the sequence listing.

Primer set for the Enol gene promoter
K24 (Fw): TTCGCGGCCGCGGCACGCAGCGCGCAGG (SEQ ID No. 8)
K25 (Rev): TTCACTAGTTGTCTGTAGGGGAAAAAAAAAAAAC (SEQ ID No. 9)

### (Example 2) Evaluation of the Chinese hamster-derived Enol gene promoter by fed-batch culture with antibody expression levels as indicator

### (2-1) Construction of antibody expression vector

As an antibody expression vector, pDSLH3.1-Eno1-Y was constructed by substituting a nucleotide sequence corresponding to the Hspa5 gene promoter of humanized antibody gene Y expression vector pDSLH3.1-Hspa5-Y described in Patent Literature 4 with the nucleotide sequence of the Chinese hamster-derived Enol gene promoter using the DNA fragment amplified and purified in Example 1. The vectors are schematically shown in Figure 1. pDSLH3.1-hEF1α-Y used for comparison is as described in Patent Literature 4.

### (2-2) Generation of a humanized antibody Y-expressing stable pool

CHO-K1 cells (ATCC) were adapted to suspension culture in a serum-free medium, to obtain CHO-O1 cells as host cells. The CHO-O1 cells were transfected with the antibody expression vector pDSLH3.1-Eno1-Y constructed in (2-1) or pDSLH3.1-hEF1α-Y described in Patent Literature 4 using a transfection apparatus Neon Transfection System (Invitrogen), and cultured in 5% CO₂ at 37°C in a T-25 flask. One day after the transfection, Geneticin (Life Technologies Corporation) was added thereto at a final concentration of 800 µg/mL, followed by a drug selection culture for 1 week. Then, the cells were cultured in 5% CO₂ at 37°C in a 125 mL Erlenmeyer flask to obtain a humanized antibody Y-expressing stable pool (two pools for each vector).

### (2-3) Evaluation of the amount of antibody produced by fed-batch culture of humanized antibody Y-expressing stable pools

Fed-batch culture was performed for 14 days in a 125 mL Erlenmeyer flask using each humanized antibody Y-expressing stable pool generated in (2-2). The culture conditions were as follows: the initial seeding density is 0.3 × 10⁶ cells/mL, the amount of the culture solution is 30 mL, the basal medium used is G13 (custom medium manufactured by FUJIFILM Wako Pure Chemical Corporation), and the feed medium used is F13 (custom medium manufactured by FUJIFILM Wako Pure Chemical Corporation). The feed medium was added in an amount of 3% (v/v) of the initial culture solution amount every day from day 3 to day 13 of culture. On days 7, 10 and 14, sampling was performed, and the viable cell density, the amount of antibody produced, and the like were measured.

The change in the number of viable cells and the change in the amount of antibody produced are shown in Figures 2A and 2B, respectively. An average value of the amounts of antibodies produced in two pools generated for each vector, and the antibody productivity was compared. The results showed that the amount of antibody produced with the Enol promoter on day 14 of the culture reached 2.0 times the value of that with the human EF1-α promoter, and thus greatly exceeded the amount of antibody produced with a promoter that is currently frequently used.

### (Example 3) Study on Chinese hamster-derived Enol gene promoter length with the amount of luminescence of luciferase as an indicator in transient expression

### (3-1) Construction of luciferase expression vectors

Eno1 promoters with respective chain lengths were inserted into pGL4.10 (manufactured by Promega Corporation) to construct firefly-derived luciferase expression vectors. The chains whose length was studied were those extending to approximately 5.0 kbp (SEQ ID NO: 10), approximately 4.5 kbp (SEQ ID NO: 11), approximately 4.0 kbp (SEQ ID NO: 4), approximately 3.5 kbp (SEQ ID NO: 12), approximately 3.0 kbp (SEQ ID NO: 1), approximately 2.8 kbp (SEQ ID NO: 3), approximately 2.7 kbp (SEQ ID NO: 13), approximately 2.6 kbp (SEQ ID NO: 14) and approximately 2.5 kbp (SEQ ID NO: 15) upstream from the nucleotide immediately upstream of the nucleotide sequence corresponding to the start codon sequence in the Chinese hamster-derived Enol gene.

### (3-2) Evaluation of the amount of luminescence of luciferase in transient expression

Using Lipofectamin 2000 CD (manufactured by Thermo Fisher Scientific), CHO-O1 cells were transfected simultaneously with the firefly-derived luciferase expression vector constructed in (3-1) and a control vector pGL4.74 that expresses Renilla-derived luciferase. Twenty four hours after the transfection, the cells were centrifuged (1000 rpm, 3 min), collected in a 1.5 mL tube, and washed with PBS buffer. Then, the amount of luminescence of luciferase was measured using a Dual-Luciferase Reporter Assay System (manufactured by Promega Corporation). For each promoter length, transfection was performed with N = 2.

The value obtained by dividing the amount of luminescence of firefly-derived luciferase on pGL 4.10 by the amount of luminescence of Renilla-derived luciferase on pGL 4.74 was taken as promoter activity (Figure 3). Table 1 shows average values calculated from promoter activity values obtained with N = 2. The results show that the promoter activity obtained using any of the regions extending to approximately 5.0 kbp (SEQ ID NO: 10), approximately 4.5 kbp (SEQ ID NO: 11), approximately 4.0 kbp (SEQ ID NO: 4) and approximately 3.5 kbp (SEQ ID NO: 12) upstream of the start codon sequence from the nucleotide immediately upstream of the nucleotide sequence corresponding to the start codon sequence in the Chinese hamster-derived Enol gene was equal to or higher than 80% of the promoter activity obtained using a region extending to approximately 3.0 kbp (SEQ ID NO: 1) upstream of the start codon sequence from the nucleotide immediately upstream of the nucleotide sequence corresponding to the start codon sequence in the Chinese hamster-derived Enol gene. The promoter activity obtained using a region extending to 2.8 kbp (SEQ ID NO: 3) upstream of the start codon sequence from the nucleotide immediately upstream of the nucleotide sequence corresponding to the start codon sequence was substantially equal to the promoter activity obtained using a region extending to approximately 3.0 kbp (SEQ ID NO: 1) upstream of the start codon sequence from the nucleotide immediately upstream of the nucleotide sequence corresponding to the start codon sequence. The promoter activity obtained using any of the regions extending to 2.7 kbp (SEQ ID NO: 13) and 2.6 kbp (SEQ ID NO: 14) upstream of the start codon sequence from the nucleotide immediately upstream of the nucleotide sequence corresponding to the start codon sequence was approximately 50% of the promoter activity obtained using a region extending to approximately 3.0 kbp (SEQ ID NO: 1) upstream of the start codon sequence from the nucleotide immediately upstream of the nucleotide sequence corresponding to the start codon sequence. The promoter activity obtained using a region extending to 2.5 kbp (SEQ ID NO: 15) upstream of the start codon sequence from the nucleotide immediately upstream of the nucleotide sequence corresponding to the start codon sequence was approximately 15% of the promoter activity obtained using a region extending to approximately 3.0 kbp (SEQ ID NO: 1) upstream of the start codon sequence from the nucleotide immediately upstream of the nucleotide sequence corresponding to the start codon sequence. Thus, it was found that the transcriptional activity of the Chinese hamster-derived Enol gene promoter was markedly high in a region having a length such that it extends to approximately 2.8 kbp or more upstream of the start codon sequence from the nucleotide immediately upstream of the nucleotide sequence corresponding to the start codon sequence.

In the region between SEQ ID NO: 3 (Enol 2.8 k) and SEQ ID NO: 13 (Enol 2.7 k), the transcriptional activity of the promoter was markedly higher in SEQ ID NO: 3 (Enol 2.8 k). For this reason, it was considered that when the promoter had a differential sequence between SEQ ID NO: 18 (differential sequence between SEQ ID NO: 3 (Enol 2.8 k) and SEQ ID NO: 13 (Enol 2.7 k) (100 bp)), its transcriptional activity was particularly improved.

**[Table 1]**

| Promoter | Promoter Activity (Firefly Luc/Renilla Luc) |
|---|---|
| Enol 5k | 1.1×10² |
| Enol 4.5k | 1.0×10² |
| Enol 4k | 93 |
| Enol 3.5k | 88 |
| Enol 3.0k | 1.1×10² |
| Enol 2.8k | 1.0×10² |
| Enol 2.7k | 56 |
| Enol 2.6k | 48 |
| Enol 2.5k | 16 |

### (Example 4) Evaluation of the mouse-derived Enol gene promoter by fed-batch culture with antibody expression levels as indicator

### (4-1) Construction of antibody expression vector

pDSLH3.1-mEno1-Y, pDSLH3.1-mEno1-3.5-Y, and pDSLH3.1-mEno1-4.0-Y were constructed by substituting the promoter for antibody H chain and L chain genes in the humanized antibody gene Y expression vector pDSLH3.1-hEF1α-Y with the mouse Enol promoter. For the mouse-derived Enol gene promoter, a region extending to approximately 3.0 kbp (SEQ ID NO: 2), approximately 3.5 kbp (SEQ ID NO: 17) or approximately 4.0 kbp (SEQ ID NO: 16) upstream of the start codon sequence from the nucleotide immediately upstream of the nucleotide sequence corresponding to the start codon sequence in Enol.

Similarly, regions extending to approximately 4.0 kbp (SEQ ID NO: 4), approximately 3.5 kbp (SEQ ID NO: 12) and approximately 2.8 kbp (SEQ ID NO: 3) upstream of the start codon sequence from the nucleotide immediately upstream of the nucleotide sequence corresponding to the start codon sequence in the Chinese hamster-derived Enol gene promoter were used as the Enol gene promoter to construct pDSLH3.1-Eno1-4.0-Y, pDSLH3.1-Eno1-3.5-Y and pDSLH3.1-Eno1-2.8-Y, respectively.

### (4-2) Generation of humanized antibody Y-expressing stable pools

The CHO-O1 cells were transfected with pDSLH3.1-hEF1α-Y (Patent Literature 4), DSLH3.1-Eno1-Y constructed in (2-1), and pDSLH3.1-mEno1-Y, pDSLH3.1-mEno1-3.5-Y, pDSLH3.1-mEno1-4.0-Y, pDSLH3.1-Eno1-2.8-Y, pDSLH3.1-Eno1-3.5-Y and pDSLH3.1-Eno1-4.0-Y constructed in (4-1) by the method described in 2-2). Drug selection culture was performed to generate a humanized antibody Y-expressing stable pool (two pools for each vector).

### (4-3) Evaluation of the amount of antibody produced by fed-batch culture of humanized antibody Y-expressing stable pools

Fed-batch culture was performed in a 125 mL Erlenmeyer flask using each humanized antibody Y-expressing stable pool generated in (4-2). The basal medium used was G13, and the feed medium used was F13.

The change in the number of viable cells and the change in the amount of antibody produced are shown in Figures 4-A and 4-B, respectively. An average value of the amounts of antibody produced in two pools generated for each vector, and the antibody productivities were compared. The results showed that the amounts of antibody produced with the mouse-derived Enol gene promoters 3.0 kbp (mEno1 3k), 3.5 kbp (mEno1 3.5k) and 4.0 kbp (mEno1 4k) on day 14 of culture reached, respectively, 1.3 times, 1.8 times and 1.9 times the value of that with the human EF1**α** promoter (hEF1**α**), and thus greatly exceeded the amount of antibody produced with a promoter that is currently frequently used. It was found that the mouse-derived Enol gene promoter was equivalent in promoter activity to the Chinese hamster-derived Enol gene promoter.

The amounts of antibody produced with the Chinese hamster-derived Enol gene promoters 2.8 kbp (Enol 2.8k), 3.0 kbp (Enol 3k), 3.5 kbp (Enol 3.5k) and 4.0 kbp (Enol 4k) reached, respectively, 2.7 times, 2.0 times, 2.8 times and 3.1 times the value of that with the human EF1**α** promoter (on day 14 of culture), and thus greatly exceeded the amount of antibody produced with a promoter that is currently frequently used.

It was found that the Chinese hamster-derived Enol gene promoter was superior in antibody productivity to the mouse-derived Enol gene promoter.

The amount of antibody produced per cell per day (Qp) was calculated from the following equation.
Qp = [amount of antibody produced on day 10] ÷ [integral viable cell density on day 10] (the "integral viable cell density on day 10" is an integral value of time-dependent changes in viable cell density, which were calculated using the viable cell density on days 0, 7 and 10).

The amounts of antibody produced with the Chinese hamster-derived Enol gene promoters 2.8 kbp (Enol 2.8k), 3.0 kbp (Enol 3k), 3.5 kbp (Enol 3.5k) and 4.0 kbp (Enol 4k) reached, respectively, 2.7 times, 2.3 times, 3.0 times and 3.1 times the value of that with the human EF1**α** promoter.

The amounts of antibody produced with the mouse-derived Enol gene promoters 3 kbp (mEno1 3k), 3.5 kbp (mEno1 3.5k) and 4k (mEno1 4k) reached, respectively, 1.9 times, 2.2 times and 2.4 times the value of that with the human EF1**α** promoter.

It was found that the Chinese hamster-derived Enol gene promoter was also superior in the amount of antibody produced per cell (Qp) to the mouse-derived Enol gene promoter.

### (Example 5) Study on effect brought about by combination of the Enol gene promoter derived from each species and A7 with antibody expression levels as indicator in fed-batch culture

### (5-1) Construction of antibody expression vector

pDSLH3.1-Eno1-Y constructed in (2-1), and pDSLH3.1-Eno1-3.5-Y, pDSLH3.1-Eno1-2.8-Y, pDSLH3.1-mEno1-Y, pDSLH3.1-mEno1-3.5-Y, pDSLH3.1-mEno1-4.0-Y and pDSLH3.1-Eno1-4.0-Y constructed in (4-1) were used as parent vectors. The DNA element A7 (SEQ ID NO: 6) was inserted upstream of the expression cassettes of the vectors by a method similar to that in Patent Literature 5 to construct antibody expression vectors.

### (5-2) Generation of humanized antibody Y-expressing stable pools

The CHO-O1 cells were transfected with the parent vector and the antibody expression vector A7 according to the method described in (2-2). Drug selection culture was performed to generate a humanized antibody Y-expressing stable pool (two pools for each vector).

### 5-3) Evaluation of the amount of antibody produced by fed-batch culture of humanized antibody Y-expressing stable pools

Fed-batch culture was performed in a 125 mL Erlenmeyer flask using each humanized antibody Y-expressing stable pool generated in (5-2). The basal medium used was G13, and the feed medium used was F13. The change in the number of viable cells and the change in the amount of antibody produced are shown in Figures 5A and 5B, respectively. An average value of the amounts of antibodies produced in two pools generated for each vector, and the antibody productivities were compared. On day 14 of culture, the amount of antibody produced from the A7-containing antibody expression vector was 1.7 to 5.4 times the value of that from the A7-free antibody expression vector. Accordingly, it was found that either the Chinese hamster-derived or mouse-derived Enol gene promoter had greatly improved antibody productivity when used in combination with the DNA element A7.

### Sequence Listing Free Text

SEQ ID NO: 1 - Nucleotide sequence of the Chinese hamster-derived Enol gene promoter from a nucleotide approximately 3.0 kbp upstream of the start codon of the Enol gene to the nucleotide immediately upstream of the nucleotide sequence corresponding to the start codon
SEQ ID NO: 2 - Nucleotide sequence of the Mouse-derived Enol gene promoter from a nucleotide approximately 3.0 kbp upstream of the start codon of the Enol gene to the nucleotide immediately upstream of the nucleotide sequence corresponding to the start codon
SEQ ID NO: 3 - Nucleotide sequence of the Chinese hamster-derived Enol gene promoter from a nucleotide approximately 2.8 kbp upstream of the start codon of Enol gene to the nucleotide immediately upstream of the nucleotide sequence corresponding to the start codon
SEQ ID NO: 4 - Nucleotide sequence of Chinese hamster-derived Enol gene promoter from a nucleotide approximately 4.0 kbp upstream of the start codon of Enol gene to the nucleotide immediately upstream of the nucleotide sequence corresponding to the start codon
SEQ ID NO: 5 - Nucleotide sequence of DNA element A2
SEQ ID NO: 6 - Nucleotide sequence of DNA element A7
SEQ ID NO: 7 - Nucleotide sequence of DNA element A18
SEQ ID NO: 8 - Primer for Enol gene promoter K24
SEQ ID NO: 9 - Primer for Enol gene promoter K25
SEQ ID NO: 10 - Nucleotide sequence of the Chinese hamster-derived Enol gene promoter from a nucleotide approximately 5.0 kbp upstream of the start codon of the Enol gene to the nucleotide immediately upstream of the nucleotide sequence corresponding to the start codon
SEQ ID NO: 11 - Nucleotide sequence of the Chinese hamster-derived Enol gene promoter from a nucleotide approximately 4.5 kbp upstream of the start codon of the Enol gene to the nucleotide immediately upstream of the nucleotide sequence corresponding to the start codon
SEQ ID **NO:** 12 - Nucleotide sequence of the Chinese hamster-derived Enol gene promoter from a nucleotide approximately **3.5** kbp upstream of the start codon of the Enol gene to the nucleotide immediately upstream of the nucleotide sequence corresponding to the start codon
SEQ ID **NO:** 13 - Nucleotide sequence of the Chinese hamster-derived Enol gene promoter from a nucleotide approximately **2.7** kbp upstream of the start codon of the Enol gene to the nucleotide immediately upstream of the nucleotide sequence corresponding to the start codon
SEQ ID NO: 14 - Nucleotide sequence of the Chinese hamster-derived Enol gene promoter from a nucleotide approximately 2.6 kbp upstream of the start codon of the Enol gene to the nucleotide immediately upstream of the nucleotide sequence corresponding to the start codon
SEQ ID NO: 15 - Nucleotide sequence of the Chinese hamster-derived Enol gene promoter from a nucleotide approximately 2.5 kbp upstream of the start codon of the Enol gene to the nucleotide immediately upstream of the nucleotide sequence corresponding to the start codon
SEQ ID NO: 16 - Nucleotide sequence of the mouse-derived Enol gene promoter from a nucleotide approximately 4.0 kbp upstream of the start codon of the Enol gene to the nucleotide immediately upstream of the nucleotide sequence corresponding to the start codon
SEQ ID NO: 17 - Nucleotide sequence of the mouse-derived Enol gene promoter from a nucleotide approximately 3.5 kbp upstream of the start codon of the Enol gene to the nucleotide immediately upstream of the nucleotide sequence corresponding to the start codon
SEQ ID NO: 18 - Differential sequence between SEQ ID NO: 3 (Enol 2.8 k) and SEQ ID NO: 13 (Enol 2.7 k)

## Claims

1. A polynucleotide that is a Chinese hamster-derived gene promoter, the polynucleotide comprising a nucleotide sequence that is selected from SEQ ID NO: 1, SEQ ID NO: 3, SEQ ID NO: 4, SEQ ID NO: 10, SEQ ID NO: 11, SEQ ID NO: 12, SEQ ID NO: 13 and SEQ ID NO: 14, or has 85% or higher sequence identity to each of the sequences, or a partial sequence of the nucleotide sequence.

2. The polynucleotide according to claim 1, which comprises a nucleotide sequence that is selected from SEQ ID NO: 1, SEQ ID NO: 3, SEQ ID NO: 4, SEQ ID NO: 10, SEQ ID NO: 11 and SEQ ID NO: 12, or has 85% or higher sequence identity to each of the sequences, or a partial sequence of the nucleotide sequence.

3. The polynucleotide according to claim 1, which consists of a nucleotide sequence having 99% or higher sequence identity to the nucleotide sequence of SEQ ID NO: 1.

4. The polynucleotide according to claim 1, which consists of a nucleotide sequence having 99% or higher sequence identity to the nucleotide sequence of SEQ ID NO: 3.

5. The polynucleotide according to claim 1, which consists of a nucleotide sequence having 99% or higher sequence identity to the nucleotide sequence of SEQ ID NO: 4.

6. The polynucleotide according to claim 1, which consists of a nucleotide sequence having 99% or higher sequence identity to the nucleotide sequence of SEQ ID NO: 10.

7. The polynucleotide according to claim 1, which consists of a nucleotide sequence having 99% or higher sequence identity to the nucleotide sequence of SEQ ID NO: 11.

8. The polynucleotide according to claim 1, which consists of a nucleotide sequence having 99% or higher sequence identity to the nucleotide sequence of SEQ ID NO: 12.

9. The polynucleotide according to claim 1, which consists of a nucleotide sequence having 99% or higher sequence identity to the nucleotide sequence of SEQ ID NO: 13.

10. The polynucleotide according to claim 1, which consists of a nucleotide sequence having 99% or higher sequence identity to the nucleotide sequence of SEQ ID NO: 14.

11. A polynucleotide that is a mouse-derived gene promoter, the polynucleotide comprising a nucleotide sequence that is selected from SEQ ID NO: 2, SEQ ID NO: 17 and SEQ ID NO: 16, or has 85% or higher sequence identity to each of the sequences, or a partial sequence of the nucleotide sequence.

12. The polynucleotide according to claim 11, which consists of a nucleotide sequence having 99% or higher sequence identity to the nucleotide sequence of SEQ ID NO: 2.

13. The polynucleotide according to claim 11, which consists of a nucleotide sequence having 99% or higher sequence identity to the nucleotide sequence of SEQ ID NO: 17.

14. The polynucleotide according to claim 11, which consists of a nucleotide sequence having 99% or higher sequence identity to the nucleotide sequence of SEQ ID NO: 16.

15. A polynucleotide comprising a nucleotide sequence having 90% or higher sequence identity to the nucleotide sequence of SEQ ID NO: 1, SEQ ID NO: 3, SEQ ID NO: 4, SEQ ID NO: 10, SEQ ID NO: 11, SEQ ID NO: 12, SEQ ID NO: 13, SEQ ID NO: 14, SEQ ID NO: 2, SEQ ID NO: 17 or SEQ ID NO: 16 according to claim 1 or 11, or a partial sequence of the nucleotide sequence, the polynucleotide having promoter activity.

16. A polynucleotide comprising a nucleotide sequence having 95% or higher sequence identity to the nucleotide sequence of SEQ ID NO: 1, SEQ ID NO: 3, SEQ ID NO: 4, SEQ ID NO: 10, SEQ ID NO: 11, SEQ ID NO: 12, SEQ ID NO: 13, SEQ ID NO: 14, SEQ ID NO: 2, SEQ ID NO: 17 or SEQ ID NO: 16 according to claim 1 or 11, or a partial sequence of the nucleotide sequence, the polynucleotide having promoter activity.

17. A polynucleotide that hybridizes under stringent conditions to a polynucleotide consisting of a nucleotide sequence complementary to the nucleotide sequence of SEQ ID NO: 1, SEQ ID NO: 3, SEQ ID NO: 4, SEQ ID NO: 10, SEQ ID NO: 11, SEQ ID NO: 12, SEQ ID NO: 13, SEQ ID NO: 14, SEQ ID NO: 2, SEQ ID NO: 17 or SEQ ID NO: 16 according to claim 1 or 11, the polynucleotide having promoter activity.

18. The polynucleotide according to claim 17, which is a Chinese hamster-derived gene promoter and comprises a nucleotide sequence of SEQ ID NO: 18, or a nucleotide sequence having 85% or higher sequence identity to the sequence.

19. A foreign gene expression unit comprising the polynucleotide according to any one of claims 1 to 18 and a foreign gene.

20. The foreign gene expression unit according to claim 19, wherein the foreign gene is a gene encoding a protein.

21. The foreign gene expression unit according to claim 20, wherein the foreign gene is a gene encoding a heteromultimeric protein.

22. The foreign gene expression unit according to claim 21, wherein the foreign gene is a gene encoding an antibody or an antigen-binding fragment thereof.

23. A foreign gene expression vector comprising the foreign gene expression unit according to any one of claims 19 to 22.

24. A foreign gene expression vector comprising the foreign gene expression unit according to any one of claims 19 to 22, and one or more polynucleotides selected from the following (a) to (e):
(a) a polynucleotide comprising the nucleotide sequence of SEQ ID NO: 5, or a partial sequence thereof;
(b) a polynucleotide comprising the nucleotide sequence of SEQ ID NO: 6, or a partial sequence thereof;
(c) a polynucleotide comprising the nucleotide sequence of SEQ ID NO: 7, or a partial sequence thereof;
(d) a polynucleotide comprising a nucleotide sequence having 80% or higher sequence identity to the nucleotide sequence of SEQ ID NO: 5, SEQ ID NO: 6 or SEQ ID NO: 7, or a partial sequence of the nucleotide sequence, the polynucleotide having foreign gene expression-enhancing activity; and
(e) a polynucleotide comprising a nucleotide sequence having 85% or higher sequence identity to the nucleotide sequence of SEQ ID NO: 5, SEQ ID NO: 6 or SEQ ID NO: 7, or a partial sequence of the nucleotide sequence, the polynucleotide having foreign gene expression-enhancing activity.

25. A transfected cell into which the foreign gene expression vector according to claim 23 or 24 has been introduced.

26. The transfected cell according to claim 25, wherein the cell is a cultured cell derived from a mammal.

27. The transfected cell according to claim 26, wherein the cell is a COS-1 cell, a 293 cell, or a CHO cell.

28. A method for producing a foreign gene-derived protein, comprising culturing the transfected cell according to any one of claims 25 to 27, and obtaining the foreign gene-derived protein from the culture.

29. The production method according to claim 28, wherein the foreign gene-derived protein is a monomeric protein or a multimeric protein.

30. The production method according to claim 29, wherein the multimeric protein is a heteromultimeric protein.

31. The production method according to claim 30, wherein the heteromultimeric protein is an antibody protein.

32. Use of the polynucleotide according to any one of claims 1 to 18 for expressing a foreign gene in a transfected cell.

33. Use of the foreign gene expression vector according to claim 23 or 24 for expressing a foreign gene in a transfected cell.
